**Europäisches Patentamt**

**European Patent Office**

**Office européen des brevets**

(11) Numéro de publication: **0 206 849 B1**

## FASCICULE DE BREVET EUROPEEN

(45) Date de publication de fascicule du brevet: **29.09.93**

(51) Int. Cl.5: **G01N 33/574**, G01N 33/577, C07K 15/06, C12N 5/00, C12N 15/00, C12P 21/00, C12Q 1/68

(21) Numéro de dépôt: **86400957.6**

(22) Date de dépôt: **30.04.86**

Demande divisionnaire 92105747.7 déposée le 30/04/86.

Le dossier contient des informations techniques présentées postérieurement au dépôt de la demande et ne figurant pas dans le présent fascicule.

(54) **Moyens pour le diagnostic in vitro de cellules malignes originaires du tube digestif.**

(30) Priorité: **02.05.85 FR 8506707**
**13.11.85 FR 8516820**

(43) Date de publication de la demande:
**30.12.86 Bulletin 86/52**

(45) Mention de la délivrance du brevet:
**29.09.93 Bulletin 93/39**

(84) Etats contractants désignés:
**AT BE CH DE FR GB IT LI LU NL SE**

(56) Documents cités:

**CHEMICAL ABSTRACTS, vol. 98, no. 9, 28 fevrier 1983, Columbus, OH (US); D.DRENCKHAHN et al., p. 244, no. 67511j***

**CHEMICAL ABSTRACTS, vol. 100, no. 23, 04 juin 1984, Columbus, OH (US); D.A.AUSIELLO et al., p. 348, no. 189079e***

(73) Titulaire: **INSTITUT PASTEUR**
**25/28, rue du Docteur Roux**
**F-75015 Paris(FR)**

Titulaire: **CENTRE NATIONAL DE LA RECHER-CHE SCIENTIFIOUE (CNRS)**
**15, Ouai Anatole France**
**F-75700 Paris Cedex 07(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Arpin, Monique**
**9, rue Roli**
**F-75014 Paris(FR)**
Inventeur: **Pringault, Eric**
**26, rue au Maire**
**F-75003 Paris(FR)**

CHEMICAL ABSTRACTS, vol. 95, no. 13, 28 septembre 1981, Columbus, OH (US); J.R.GLENNEY Jr. et al., p. 249, no. 110516s*

PASCAL, 1984, Centre de Documentation Scientifique et Technique du CNRS, Paris (FR); E.COUDRIER et al., no. 84-X-0100064*

PROCEEDINGS OF THE NATL. ACADEMY OF SCIENCES USA, vol. 82, no. 20, octobre 1985; P.MATSUDAIRA et al., pp. 6788-6792*

PROCEEDINGS OF THE NATL. ACADEMY OF SCIENCES USA, vol. 82, no. 24, décembre 1985; S.ROBINE et al., pp. 8488-8492*

J. SUBMICROSC. CYTOL., vol. 16, no. 1, 1984; COUDRIER et al., pp. 159-160*

Inventeur: **Garcia, Alphonse**
**3, rue de Viroflay**
**F-75015 Paris(FR)**
Inventeur: **Louvard, Daniel**
**23, Allée de Trévise**
**F-92330 Sceaux(FR)**
Inventeur: **Dudouet, Brigitte**
**9, rue Blainville**
**F-75005 Paris(FR)**
Inventeur: **Robine, Sylvie**
**54, rue d'Issy**
**F-92170 Vanves(FR)**

(74) Mandataire: **Desaix, Anne et al**
**Ernest Gutmann - Yves Plasseraud S.A., 67 bld.Haussmann**
**F-75008 Paris (FR)**

## Description

L'invention est relative à des moyens de diagnostic in vitro de l'existence ou non chez l'homme des proliférations de cellules malignes à partir de cellules tumorales du tube digestif, plus particulièrement du type adénocarcinome. Ces moyens concernent tant des procédés de diagnostic in vitro, que des éléments matériels susceptibles d'être mis en oeuvre dans ces procédés, plus particulièrement des fragments d'ADN utilisables comme sondes ou encore des anticorps, de préférence monoclonaux, dirigés contre la villine humaine.

L'invention étend encore ses effets aux différentes applications de ces anticorps monoclonaux, par exemple à la purification de la villine humaine à partir d'extraits cellulaires la contenant. A ce titre, l'invention concerne aussi la villine humaine purifiée elle-même.

Il a déjà eté proposé d'avoir recours a la détection de protéines de structure en tant que marqueurs spécifiques, pour l'identification de certains types de cellules d'eucaryotes supérieurs et pour l'étude du suivi des différentes étapes de leur developpement, notamment au cours de leur différenciation. A titre d'exemples de marqueurs connus, on mentionnera des protéines de filaments, par exemple la vimentine, des keratines ou des protéines de neurofilaments, qui ont permis l'identification de certains types de cellules, l'étude de leur développement, à partir du stade de l'embryogenèse jusqu'au stade ultime de la différenciation cellulaire, ainsi que de leur comportement vis-à-vis du milieu environnant (E. LAZARIDES, Nature, 283, 249 (1980) et R. MOLL et al., Cell 31, 11 (1982).

Les kératines sont souvent considérées comme des marqueurs particulièrement efficaces, permettant de distinguer les cellules épithéliales et les cellules non épithéliales. Ceci étant, il a été établi que ce type de distinction ne permettait pas toujours d'établir de façon non ambiguë l'origine des cellules épithéliales en cause.

Pourtant la nécessité d'établir de façon certaine l'origine de certains types de cellules revêt une importance particulièrement importante dans un grand nombre de cas, par exemple lorsqu'il s'agit, à l'occasion de l'examen d'un prélèvement biologique témoignant d'une prolifération de cellules tumorales d'identifier les cellules tumorales primaires, se trouvant à l'origine de cette prolifération, ou encore de détecter l'existence de métastases dont l'origine serait un cancer de la région digestive ou encore de vérifier l'efficacité d'un traitement chimiothérapeutique ou autres contre des métastases cancéreuses. On sait bien que l'efficacité d'un traitement anticancéreux peut être liée à l'identification précise des cellules tumorales qui sont la cause première de la maladie.

Ces observations s'appliquent avec une force particulière à la détection, de préférence précoce, de proliférations de cellules tumorales d'origine digestive (responsables d'une importante proportion des cancers observés en clinique) ou rénales, ou de cellules dérivées des cellules précédentes, plus particulièrement à l'identification de marqueurs caractéristiques de ces cellules.

L'invention s'intéresse donc plus particulièrement à la détection de cellules tumorales qui ont conservé certains des phénotypes essentiels des cellules épithéliales normalement présentes dans les muqueuses de l'appareil digestif, plus particulièrement les muqueuses intestinales, et dont les types différenciés majeurs sont constitués par les cellules absorbantes ou entérocytes.

Plus particulièrement encore l'invention se rapporte à des marqueurs spécifiques susceptibles d'être détectés, qu'ils soient ou non portes par ces cellules tumorales.

En particulier l'invention vise à permettre la détection de ces marqueurs, même lorsqu'ils sont détachés desdites cellules tumorales, notamment à l'occasion de la nécrose de ces cellules, et libérés, par exemple dans la circulation sanguine.

Le marqueur mis en jeu dans le cadre de la présente invention est constitué par tout ou partie de la villine. Il s'agit d'une protéine ayant un poids moléculaire de l'ordre de 95.000 daltons et qui est normalement presente dans les villosités des muqueuses digestives, plus particulièrement intestinales. La villine est capable de se lier a l'actine, en présence d'ions calcium.

Dans un article publié en 1984 (J. Submicrosc. Cytol. 16 (1): 159-160) Coudrier et al ont décrit à partir de l'observation d'une lignée cellulaire de carcinome, la présence de villine dans des cellules différenciées et dans des cellules indifférenciées. La villine a été détectée dans cette lignée cellulaire, au moyen d'anticorps polyclonaux.

D'une espèce à l'autre, la séquence en acides aminés de la villine peut légèrement différer, mais les caractéristiques fonctionnelles sont maintenues. Les techniques de purification connues à ce jour permettent d'obtenir à l'état pratiquement pur la villine de poulet et celle de porc. En revanche ces techniques ne conduisent pas à l'obtention de villine humaine purifiée.

L'une des villines les mieux connues est celle du poulet. Elle est formée d'une séquence de 854 acides aminés. Elle peut être coupée par protéolyse ménagée avec la trypsine et la protéase V-8 en des points déterminés, pour produire des fragments 44 Kd, 51 Kd et 10 Kd dont les positions relatives apparaissent dans la carte, de la villine, d'après Paul MADSUDAIRA, MRC, Laboratory of Molecular Biology, Cambridge, U.K. et John GLENNEY et al, J.B.C., 1981, 256, 8156-8161, qui s'établit comme suit :

```
Fragments protéolyse ménagée        Site de liaison à
        /              \            l'actine, indépen-
       /                \           dante de la présence
      /                  \          de calcium
      ↓                   ↓
   44 Kd               51 Kd        V8      10 Kd (HP)
             Ti
              ↓                      ↓
NH₂ ├────────────────────────────────────────────┤ COOH

      1 ├──┤ 69                    779 ├──────────┤ 854
   site de liaison à              région connue quant
   l'actine, dépendant            à la séquence en
   de la présence de calcium      acides aminés
```

Ti : rupture trypsique
V8 : rupture avec la protéase V8.

La séquence en acides aminés de la partie COOH-terminale (779-854) ou "partie de tête" (head piece ou "peptide HP") pour la villine de poulet est la suivante :

```
      1                                               10  11
   Val-Phe-Thr-Ala-Thr-Thr-Thr-Leu-Val-Pro-Thr-Lys-Leu-Glu-
                       20
   Thr-Phe-Pro-Leu-Asp-Val-Leu-Val-Asn-Thr-Ala-Ala-Glu-Asp-
        30  31                                    40  41
   Leu-Pro-Arg-Gly-Val-Asp-Pro-Ser-Arg-Lys-Glu-Asn-His-Leu-
                       50  51
   Ser-Asp-Glu-Asp-Phe-Lys-Ala-Val-Phe-Gly-Met-Thr-Arg-Ser-
        60  61                                    70  71
   Ala-Asn-Leu-Pro-Leu-Trp-Lys-Gln-Gln-Asn-Leu-Lys-Lys-Glu-

   Glu-Lys-Gly-Leu-Phe
```

D'une manière générale, la villine est présente plus particulièrement dans les bordures en brosses qui sont observées à la surface des entérocytes, notamment lorsque ceux-ci ont atteint leur stade ultime de différentiation, au niveau des parois de la lumière intestinale. Les microvillosités sont des organelles assemblées dans le stade final de la différentiation des entérocytes. La villine est plus particulièrement localisée dans les faisceaux de microfilaments axiaux de ces microvillosités. Elle contribue à la constitution de leur cytosquelette. Par examen de coupes congelées en immunofluorescence, il a pu être constaté que la villine était présente au pôle apical des cellules allongées formant des colonnes affleurant à la paroi interne de l'intestin, et également à proximité des cellules tubulaires proximales du rein. Dans les deux cas il s'agit de régions coïncidant avec la bordure en brosse des cellules concernées. Mais la villine n'a pas été détectée au niveau de divers autres types de villosités de divers autres épithéliums, lorsque celles-ci étaient dépourvues d'une bordure en brosse très organisée (A. BRETSCHER et al, Exp. Cell. res., 135, 213 (1981) ou encore article de H. REGGIO et al, publié dans l'ouvrage intitulé "Membranes in Growth and Development" (Membranes en cours de croissance et de développement) de A. LISS, New York ((1982) pp. 89-105).

Il a en outre été constaté que la villine était présente dans les entérocytes dans un stade précoce de leur développement, bien avant la mise en place de la bordure en brosse.

L'invention résulte d'une double découverte. La villine reste détectable de façon pratiquement systématique au cours de la tumorigénèse des tissus du tube digestif, et plus particulièrement dans les cancers du colon, l'une des formes de cancers parmi les plus répandues et aussi dans certains types de cancers rénaux, alors que la villine n'a jamais été détectée dans des cellules tumorales primaires résultant de la cancérisation initiale de tissus localisés dans d'autres organes (foie, ovaires, poumons, etc.).

En outre, la villine est détectable in vivo à tous les niveaux du développement des cellules tumorales de la région digestive, tant au stade précoce de la tumorigénèse qu'aux stades les plus tardifs, quel que soit l'état de différenciation des cellules en cause.

Le procédé de diagnostic selon l'invention, que a pour but la détection in vitro de cellules tumorales initialement formées dans la région digestive et/ou dérivées de celles-ci dans un prélèvement biologique est caractérisé par la mise en contact de ce prélèvement biologique avec des réactifs présentant une affinité spécifique pour la villine ou reconnaissant le gêne codant pour cette protéine.

Le procédé de diagnostic selon l'invention est applicable à tout échantillon biologique susceptible de contenir des cellules tumorales d'origine digestive ou des cellules tumorales éventuellement formées dans d'autres régions de l'organisme, sous l'influence de cellules primaires d'origine digestive ou rénale, ou encore à tout échantillon biologique susceptible de contenir des produits de dégradation de ces diverses cellules tumorales, y compris le marqueur villine, à l'occasion de la nécrose desdites cellules. Cet échantillon biologique peut consister en toutes formes de prélèvements : fragments de tissus ou de tumeurs solides, par exemple obtenus par biopsie, ou encore prélèvement de liquides biologiques, plus particulièrement de sang total ou de sérum sanguin, susceptible de vehiculer des cellules malignes ou des fragments originaires de celles-ci.

Selon un mode de réalisation de l'invention, le réactif mis en oeuvre dans le procédé de diagnostic de l'invention est constitué par un ADN dont la séquence comporte une région codant pour une séquence d'acides aminés de la villine humaine, plus spécialement la partie COOH-terminale.

Selon un autre mode de réalisation de l'invention, les techniques mises en oeuvre dans le procédé de diagnostic font appel à des techniques immunologiques, le réactif présentant une affinité spécifique pour la villine étant alors constitué par des anticorps susceptibles de reconnaitre la villine.

Ces anticorps sont eux-mêmes marqués de toute façon en soi connue, ou sont reconnaissables à leur tour, lorsqu'ils sont à l'état fixé sur la villine ou les tissus la portant, par des immunoglobulines marquées ou autres protéines marquées (par exemple la protéine A de Staphylococcus aureus).

Des techniques particulièrement appropriées sont celles qui ont été mises en oeuvre dans les essais qui seront décrits plus loin, et qui ont conduit aux constatations exposées ci-dessus.

L'invention a également pour but de fournir des moyens nouveaux spécifiques, pour la détection de la villine au niveau des microvillosités des entérocytes ou cellules analogues (étant entendu que l'utilisation de ces moyens nouveaux n'est pas necessairement limitée à la mise en oeuvre du procédé de diagnostic conforme à l'invention).

Selon un aspect de l'invention, ces moyens sont constitués par l'ADN correspondant à l'ARNm complet de la villine humaine ou par un fragment d'ADN dont la sequence nucléotidique comporte une région codant pour une séquence d'acides aminés de la villine humaine, capable de s'hybrider spécifiquement avec une séquence nucléotidique codant pour la villine.

L'invention vise plus spécialement l'utilisation dans le procédé ci-dessus d'un ADN correspondant à l'ARNm complet de la villine humaine ou d'un fragment d'ADNc caractérisé par le fait qu'il renferme au moins une partie de la séquence nucléotidique codant pour les acides aminés de l'extrémité COOH de la villine humaine. Cette séquence nucléotidique et la séquence correspondante des acides aminés codés sont indiquées ci-après :

EP 0 206 849 B1

```
LYS  TRP SER ASN THR LYS SER TYR GLU ASP LEU LYS ALA GLU
AAG  TGG AGT AAC ACC AAA TCC TAT GAG GAC CTG AAG GCG GAG


SER GLY ASN SER ARG ASP TRP SER GLN ILE THR ALA GLU VAL
TCT GGC AAC TCT AGG GAC TGG AGC CAG ATC ACT GCT GAG GTC


THR SER PRO LYS VAL ASP VAL PHE ASN ALA ASN SER ASN LEU
ACA AGC CCC AAA GTG GAC GTG TTC AAT GCT AAC AGC AAC CTC


SER SER GLY PRO LEU PRO ILE PHE PRO LEU GLU GLN LEU VAL
AGT TCT GGG CCT CTG CCC ATC TTC CCC CTG GAG CAG CTA GTG


ASN LYS PRO VAL GLU GLU LEU PRO GLU GLY VAL ASP PRO SER
AAC AAG CCT GTA GAG GAG CTC CCC GAG GGT GTG GAC CCC AGC


ARG LYS GLU GLU HIS LEU SER ILE GLU ASP PHE THR GLN ALA
AGG AAG GAG GAA CAC CTG TCC ATT GAA GAT TTC ACT CAG GCC


PHE GLY MET THR PRO ALA ALA PHE SER ALA LEU PRO ARG TRP
TIT GGG ATG ACT CCA GCT GCC TTC TCT GCT CTG CCT CGA TGG


LYS GLN GLN ASN LEU LYS LYS GLU LYS GLY LEU PHE *
AAG CAA CAA AAC CTC AAG AAA GAA AAA GGA CTA TTT TGA GAAG
AGTAGCTGTGGTTGTAAAGCAGTACCCTACCCTGATTGTAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAAA
```

La séquence nucléotidique mise en oeuvre est utilisée comme sonde pour la détection des ARNm ou des ADN codant pour la villine humaine.

Une sonde appropriée pour ce type de détection est avantageusement marquée par un élément radioactif ou tout autre groupe permettant sa reconnaissance à l'état hybridé avec la préparation renfermant l'ARNm ou l'ADN à étudier.

Selon les techniques classiques, ces sondes sont mises en contact avec un prélèvement biologique renfermant les cellules à étudier, ou directement avec leurs acides nucléiques, dans des conditions autorisant l'hybridation éventuelle de la séquence nucléotidique de la sonde avec une séquence complémentaire éventuellement contenue dans l'échantillon testé.

La reproductibilité de la détection a été testée en utilisant une sonde contenant un insérat de 200 paires de bases.

Ces sondes nucléotidiques, qui constituent des produits nouveaux entrant dans le cadre de l'invention, sont utilisées également, selon un autre aspect, pour l'étude de l'expression des ARNm de la villine dans chacun des états de différenciation des entérocytes et/ou des cellules exprimant la villine, et dans les différents stades de maturation des cellules d'origine rénale ou intestinale.

Les sondes obtenues permettent ainsi de déterminer le nombre, l'organisation et la structure du ou des génes de la villine tels qu'isolés à partir d'une banque génomique humaine.

6

EP 0 206 849 B1

Conformément à l'invention, on isole la séquence nucléotidique codant pour l'extrémité de la villine humaine en procédant selon les étapes suivantes :

- on prépare selon les techniques connues les ARNm (ARN messagers) totaux à partir d'une lignée cellulaire exprimant la villine ;
- on construit une banque d'ADNc (ADN complémentaires) :
- on insère les ADNc dans un vecteur susceptible d'exprimer la protéine codée par l'insérat ;
- on transforme à l'aide des vecteurs recombinants obtenus une souche bactérienne, puis on établit des conditions permettant l'expression de la protéine recherchée dans la bactérie ;
- on sélectionne les clones recombinants contenant le clone spécifique de la villine à l'aide d'anticorps reconnaissant la villine.

L'étape de construction de la banque d'ADNc est réalisée à l'aide d'un vecteur capable d'exprimer la protéine codée par l'ADNc inséré.

Des vecteurs de clonage particulièrement avantageux, en raison notamment de leur rendement d'expression élevé, sont constitués par des plasmides de type pEX. De tels plasmides sont décrits par Stanley et Luzio dans EMBO Journal, 1984, 3, 1429-1434. Ils dérivent de plasmides contenant le gène de fusion cro-lacZ dont l'expression est sous le contrôle du promoteur $P_R$ du bactériophage lambda. Cette expression est inductible par la température.

Un polynucléotide contenant plusieurs sites uniques de restriction est inséré dans chacune des phases de lecture, ainsi que les signaux de terminaison de la transcription et de la traduction. Les ADNc insérés au niveau des sites uniques de restriction sont exprimés avec une grande efficacité sous forme de protéine hybride cro-$\beta$gal-peptide correspondante. Cette protéine hybride est peu soluble, ce qui réduit d'une manière avantageuse les risques de protéolyse dans la bactérie et facilite sa détection immunologique par des anticorps spécifiques.

Pour réaliser l'insertion de l'ADNc dans la bonne orientation par rapport au gène cro-lacZ, afin d'obtenir l'expression dans la bactérie du polypeptide correspondant, on utilise un protocole d'addition séquentielle de linkers différents aux deux extrémités de l'ADNc selon la technique de Helfmann et al. dans P.N.A.S. USA, 1983, 80, 31-35. Les linkers utilisés correspondent aux sites de coupure de BamHI et SalI.

Ces plasmides recombinants sont utilisés pour transformer, selon les techniques classiques, la souche bactérienne mise en oeuvre pour l'expression de la protéine par la séquence recherchée.

Les souches bactériennes de E. coli sont particulièrement préférées, plus spécialement la souche E. coli pop 2135 qui contient l'allèle clts857 du répresseur cro.

On obtient ainsi dans la souche bactérienne une banque d'ADNc correspondant à l'ARNm des cellules exprimant un haut niveau de villine, contenant environ 30 000 clones recombinants.

L'expression de la protéine hybride est inductible par la température. On opère donc à une température où le répresseur du gène cro n'est plus actif. A cet effet, il est satisfaisant d'incuber les souches environ 2 heures à une température supérieure à l'ambiante, en particulier de l'ordre de 40-42 °C.

Les clones récupérés après lyse des bactéries et renaturation des protéines selon les techniques classiques sont sélectionnés par criblage immunologique.

D'une manière préférée, on effectue d'abord un criblage de la banque d'ADNc par au moins un type d'anticorps polyclonal anti-villine. Les clones réagissant spécifiquement avec le ou les anticorps polyclonaux sont recriblés à l'aide d'au moins un type d'anticorps monoclonal dirigé contre des épitopes COOH-terminaux.

Un criblage satisfaisant est réalisé à l'aide d'un anticorps anti-villine polyclonal tel qu'un anticorps polyclonal dirigé contre la villine de porc intacte puis un ou plusieurs anticorps monoclonaux reconnaissant des épitopes situés dans la région COOH-terminale de la molécule.

Le criblage à l'aide de l'anticorps polyclonal est avantageusement suivi d'un criblage secondaire à l'aide d'un autre anticorps polyclonal, en particulier un anticorps polyclonal dirigé contre le fragment COOH-terminal de la villine de poulet.

L'invention vise plus particulièrement un clone porteur d'un ADNc correspondant à la villine humaine caractérisé en ce qu'il réagit spécifiquement avec l'anticorps polyclonal dirigé contre la villine intacte, avec l'anticorps polyclonal dirigé contre le peptide COOH-terminal de la villine de poulet et avec les deux anticorps monoclonaux dirigés contre des épitopes COOH-terminaux.

L'invention vise également un clone porteur d'un ADNc correspondant à la villine comprenant un insérat avec une séquence polyA et un site de poly-adénylation.

Selon un autre aspect de l'invention, les moyens mis en oeuvre pour la détection de la villine sont constitués par des anticorps monoclonaux.

Cet aspect de l'invention s'appuie sur le fait que les villines, originaires de différentes espèces, possèdent des épitopes communs, accessibles à des anticorps monoclonaux reconnaissant de façon

7

EP 0 206 849 B1

spécifique non seulement la villine particulière ayant servi à l'immunisation de l'animal, dont les cellules spléniques avaient été fusionnées avec les cellules de myélome appropriées à la production des hybridomes secréteurs desdits anticorps monoclonaux, mais reconnaissant de façon aussi spécifique des villines originaires d'autres espèces, y compris l'espèce humaine.

Un anticorps monoclonal préféré selon l'invention est donc caractérisé par les propriétés suivantes :
- Il reconnaît la villine de porc purifiée (Western Blotting).
- Il reconnaît la villine de poulet (Western Blotting).
- Il reconnaît la villine d'extrait cellulaire de la lignée dérivée d'un adénocarcinome de colon humain : HT29 (Western Blotting).
- Il reconnaît la villine de rat en immunocytochimie (coupes congelées (cryostat) de muqueuse intestinale de rat fixées au formaldéhyde).

Un anticorps monoclonal préféré parmi ceux qui répondent à la définition précédente reconnaît également un épitope contenu dans le "peptide HP" de la villine de poulet, la reconnaissance étant possible tant à l'égard du peptide HP préalablement isolé qu'à l'égard du peptide 51Kd contenant encore le peptide HP ; cet anticorps ne reconnaît ni le peptide 44Kd ni le peptide 44Kd de la villine de poulet.

L'anticorps préféré de l'invention reconnaît d'ailleurs la protéine HP, aussi bien à l'état dénaturé, notamment après traitement par le dodécylsulfate de sodium (SDS) que sur les cellules épithéliales parteuses de la villine. Ce résultat démontre que l'épitope porté par le peptide HP n'est pas masqué par d'autres protéines dans le milieu cellulaire.

L'invention concerne naturellement egalement, les hybridomes secréteurs de ces anticorps monoclonaux. Une technique avantageuse pour produire ces hybridomes, notamment par fusion de cellules spléniques de souris immunisées contre la villine purifiée de porc, d'une part, et de cellules d'un myélome approprié, sera décrit plus loin. L'hybridome préféré ainsi obtenu (souche BD-D 2C3) a été déposé à la Collection Nationale des Cultures de Microorganismes (CNCM) de l'Institut Pasteur de Paris, le 29 Avril 1985, sous le n° I-440.

Selon un aspect avantageux de l'invention, l'agent immunogène utilisé pour former des anticorps peut être exprimé en grandes quantités dans la bactérie. Cet agent immunogène correspond au peptide codé par la séquence nucléotidique définie ci-dessus correspondant à l'ADN de la villine humaine ou à un fragment.

Selon les techniques classiques, on injecte le peptide à des animaux, on récupère les antisérums, puis, le cas échéant, les anticorps à partir des antisérums, par exemple par chromatographie d'affinité. La production d'anticorps selon cette variante revêt un intérêt d'autant plus grand que la région HP codée par le fragment d'ADNc définie plus haut est à la fois la région la plus immunogène et la plus spécifique de la villine.

On remarquera que la protéine codée par la séquence nucléotidique de l'ADN complémentaire de la villine ou d'un fragment de cet ADN constitue également une source d'antigènes permettant de réaliser des radioimmunoassays compétitifs (soit par la méthode ELISA directe, soit par la méthode ELISA par compétition).

Il va cependant de soi que tout autre agent immunogène contenant la même séquence immunogène peut être utilisée ; par exemple, le peptide HP lui-même, le cas échéant greffé au préalable sur une molécule porteuse, telle que la sérumalbumine bovine, en vue d'accroître ses propriétés immunogènes.

Il apparaîtra clairement à l'homme du métier qu'ayant en mains l'anticorps monoclonal préféré selon l'invention, il est à même de définir la région épitopique caractéristique du peptide HP. Pour l'identifier de façon plus précise, il pourrait notamment avoir recours a un procédé comprenant les étapes suivantes :
- synthèse d'une séquence polynucléotidique codant pour le peptide HP (ou pour la partie de peptide HP dont il est raisonnable de présumer qu'elle contient l'épitope recherché),
- linéarisation dudit plasmide défini ci-dessus contenant la séquence nucléotidique codant pour l'extrémité COOH-terminale de la villine, et ce au niveau d'un site de restriction extérieur à ladite séquence,
- émondage de façon contrôlée du plasmide linéarisé avec une enzyme exonucléolytique, telle que l'enzyme Bal 31,
- recircularisation du plasmide au moyen d'une ADN-ligase,
- transformation d'un microorganisme approprié, lui-même transformable par le plasmide correspondant et capable d'exprimer l'insérat contenu dans celui-ci,
- remise en contact des produits d'expression de ce microorganisme avec l'anticorps considéré,
le cycle d'opérations qui vient d'être défini étant répété jusqu'à la disparition de la détection dudit peptide immunogène parmi les produits d'expression dudit microorganisme transformé par le dernier plasmide recircularisé.

8

Il est possible, au terme de chacun des cycles du procédé sus-défini, notamment séquençage des parties d'extrémité du plasmide avant et après la dernière opération d'émondage, ayant conduit à la perte de capacité de reconnaissance du plasmide recircularisé intermédiaire, de situer l'épitope au niveau du peptide codé par la séquence nucléotidique éliminée au cours de cette dernière opération d'émondage. C'est dire que le fait pour l'homme du métier de disposer de l'anticorps monoclonal sus-indiqué est équivalent à la mise à sa disposition de la séquence peptidique définie chimiquement et contenant cet épitope.

L'invention concerne également la villine humaine pratiquement pure biologiquement, réagissant avec l'anticorps monoclonal préféré défini ci-dessus et ne donnant essentiellement qu'une seule bande dans des essais de migration en électrophorèse sur gel de polyacrylamide, notamment SDS-PAGE.

En effet, la villine humaine peut être extraite d'un lysat d'entérocytes humains, notamment obtenu par traitement de ces derniers avec une solution aqueuse contenant un détergent approprié, et purifiée à l'aide du susdit anticorps monoclonal. Un procédé de purification de ce type met en jeu l'anticorps monoclonal avantageusement immobilisé sur un support solide, de préférence adapté à des opérations de chromatographie d'affinité. Par exemple, l'anticorps monoclonal est fixé sur un réseau d'agarose à réticulation tridimensionnelle, commercialisé sous la marque SEPHAROSE par la société suédoise PHARMACIA A.G., par exemple par la méthode au bromure de cyanogène.

L'invention concerne donc plus particulièrement un procédé de séparation de la villine humaine caractérisé par les opérations consistant à faire passer une solution la contenant au contact d'une colonne d'affinité portant le susdit anticorps monoclonal, pour fixer sélectivement la villine humaine, puis à récupérer celle-ci par dissociation du complexe antigène-anticorps soit par tampon acide à pH 2-4 à base de glycine, soit par un tampon basique à base de méthylamine, pH 11, puis à dialyser contre un tampon d'acétate d'ammonium.

Il va de soi que l'invention concerne également des polypeptides de poids moléculaires plus faibles consistant en des fragments de villine humaine. Il apparaîtra clairement au spécialiste que les fragments pourront être obtenus par découpage de la villine humaine par des enzymes de coupure de polypeptides en des sites spécifiques. A titre d'exemple de telles protéines, on mentionnera en premier lieu la trypsine ou la protéase de Staphylococcus aureus V8, l'alphachymotrypsine, la "protéase de glande sous-maxillaires de souris" (mouse submaxillary gland protease) commercialisée par la Société BOEHRINGER, la collagénase de Vibrio alginolyticus chemovar iophagus, qui reconnaît spécifiquement lesdits peptides Gly-Pro et Gly-Ala, etc.

Il va sans dire que font alors également partie de l'invention les hybridomes et anticorps monoclonaux spécifiques d'espèce ou non qui peuvent être fabriqués à partir de la villine humaine ou de ses fragments,

D'autres caractéristiques et avantages de l'invention apparaîtront encore dans la description qui suit des conditions particulières, indiquées à titre d'exemple, de détection de villine humaine, de production et de mise en oeuvre dans des essais d'hybridomes et d'anticorps monoclonaux.

On rapporte également un procédé d'obtention d'un clone porteur d'une portion d'ADNc de la villine humaine.

EXEMPLE 1 : DETECTION DE VILLINE HUMAINE.

L'expression de la villine peut être étudiée à différents stades de la prolifération de cellules tumorales ou non originaires des régions digestives ou rénales d'une part, et a autres régions de l'organisme d'autre part. Partant des tissus concernes, ceux-ci ont été congelés, fixés et préparés dans des conditions à permettre la réalisation de coupes (cryocoupes) selon la technique décrite par BROWN et FARQUHAR, Cell. 36, 295 (1984).

Des coupes ayant des épaisseurs de 5 $\mu$m ont été obtenues à -25°C et déposées sur des lames de verre recouvertes de polylysine. Après trempage dans une solution d'un tampon constitué d'une solution saline de phosphate et contenant 0,2 % de gélatine (PBS-gélatine), les coupes ont été traitées pour détecter la villine à l'aide d'une solution contenant 50 $\mu$l d'antisérum de lapin antivilline dilué au 1/800° dans la PBS-gélatine, puis incubées pendant 25 minutes à la température ambiante dans une chambre humide.

Les coupes ont ensuite été lavées trois fois pendant 10 minutes dans la PBS-gélatine.

Des anti-IgG de lapin marqués par la rhodamine (produit par la Société néerlandaise Nordic Immunological Laboratories) ont ensuite été ajoutés et les coupes incubées en présence de ces anti-IgG à la température ambiante pendant 25 minutes. Les coupes ont ensuite été lavées de façon complète dans la PBS-gélatine, puis montées pour examen sous microscope à fluorescence (Photomicroscope ZEISS) équipé de lentille à immersion dans une huile Plan Néofluar et d'un jeu de filtres appropriés.

Les tissus témoins ou les tissus tumoraux d'autres origines ont été traités de la même manière.

Les observations suivantes ont pu être faites:

Lorsque les tissus provenaient d'adénocarcinomes du colon, 11 observations sur 12 se sont révélées positives pour ce qui est de l'expression de la villine. Des tumeurs humaines d'autres origines telles que des lymphomes, notamment lymphomes de nodules mésentériques et des types variés de carcinomes d'autres origines résultant des métastases ou non au niveau du pancréas, de l'oesophage, n'ont pas exprimé la protéine.

D'une façon générale, les observations ont montré une corrélation étroite entre l'origine intestinale primaire de tumeurs traitées et l'expression de la villine. Les nombreux essais réalisés sur des tumeurs manifestement d'origine distincte se sont pratiquement dans tous les cas révélés négatifs au niveau de l'expression de la protéine.

Il est à remarquer que dans les tissus d'origine intestinale, la villine a toujours été observée, quel que soit le degré de prolifération desdites cellules.

Les essais qui précèdent ont été réalisés avec des anticorps polyclonaux. Des résultats beaucoup plus nets peuvent être obtenus avec l'anticorps monoclonal préféré qui a fait l'objet d'un dépôt à la CNCM.

Les conditions dans lesquelles les hybridomes secréteurs de ces anticorps monoclonaux ont été isolés seront décrites ci-après à titre d'exemples. Il sera également indiqué un mode de mise en oeuvre préféré des conditions dans lesquelles le diagnostic de la présence ou non de villine peut être détecté dans des essais immunologiques du type ELISA.

EXEMPLE 2 : PREPARATION D'ANTICORPS MONOCLONAUX DIRIGES CONTRE LA VILLINE.

A) PROGRAMME D'IMMUNISATION

- Souris Balb/C o 6 à 8 semaines.
- Antigène : villine de porc purifiée (10 mg/ml) (bande homogène après électrophorèse de la protéine sur gel de polyacrylamide (10 %) en présence de SDS de poids moléculaire 95Kd) :
  . 10 mM Imidazole
  . 75 mM HCl
  . 1 mM EGTA
  . 0,1 M DTT
  . 50 % Glycérol.
- Programme :
  . Jour 0 : Injection en IP de 50 $\mu$g de villine pure dans une émulsion 50/50 (PBS-Adjuvant de Freund Complet).
  . Jour 14 : Rappel en IP 50 $\mu$g de villine (émulsion PBS-Adjuvant de Freund Incomplet).
  . Jour 21 : Rappel en IP 50 $\mu$g de villine (émulsion PBS-Adjuvant de Freund Incomplet).
  . Jour 28 : Injection en IM de 20 $\mu$g de villine pure en solution dans du PBS.
  . Jour 29 : Injection IV de 10 $\mu$g de villine pure en solution dans du PBS.
  . Jour 32 : Fusion.

B) FUSION

I) Cellules parentales
  a) Cellules de myélome :
    . Lignée Sp2/0-Ag14 (8 Azaguanine résistant).
    . Culture stérile en milieu DMEM 10 % FCS.
  b) Cellules spléniques :
    Origine : rate de souris o Balb/C hyperimmunisée contre la villine de porc.
II) Procédé de fusion cellulaire selon Kùhler G. et Milstein C. (Continuous culture of fused-cells secreting antibody of predefined specificity, Nature 1975, 256, 495).
  - Fusion en milieu DMEM sans sérum dans des conditions stériles, en présence d'une solution de polyéthylèneglycol (PEG 1000-Merck 9729) à 50 % dans un milieu DMEM sans sérum. Après dénombrement des deux types de cellules parentales, la suspension de cellules de myélome en culture est melangée a la suspension de cellules spleniques dans la proportion de 1 pour 5.
  - Contact 2′30″.
  - Arrèt de l'action du PEG par dilution en milieu DMEM complet.
  - Dilution finale de la suspension cellulaire ($2 \times 10^5$ cellules/ml) dans du milieu sélectif DMEM-HAT.

- Distribution dans puits boîte COSTAR-24 trous 1 ml/puits (COSTAR Tissue Culture Cluster 24, Cat. N° 3524, COSTAR 205 Groadway, Cambridge, Ma. USA).

III) Sélection des clones

Les clones sont identifiés pour leur capacité à sécréter des anticorps dirigés contre la villine purifiée de porc.

C) MÉTHODE DE SELECTION

I) Test ELISA : Ce test permet la mise en évidence d'anticorps monoclonaux anti-villine dans les surnageants de culture après fusion.

- Fixation de l'antigène sur support (plaque ELISA) Concentration : 5 $\mu$g/ml dans un tampon phosphate de potassium 50 mM pH 8, 1 nuit à 4°C.
- Saturation avec PBS-Tween 20-BSA.
- Incubation I avec surnageant d'hybridome non dilué, 3 heures à 4°C.
- Incubation II avec des anti-IgG de souris marquées à la bêta-galactosidase, 2 heures à 4°C.
    Tous les lavages entre chaque étape sont effectués avec du PBS-Tween 20 0,1 %.
- Substrat O-nitrophényl-bêta-D-galactopyranoside (Sigma N11-27) dans du tampon phosphate 0,1 M pH 7,0. $10^{-3}$ M MgSO$_4$. $2 \times 10^{-3}$ M MnSO$_4$. $2 \times 10^{-3}$ M magnésium Tritriplex déshydraté (Merck 8409).
    Lecture : DO 414 nm.

Les clones sélectionnés sont ceux pour lesquels les surnageants d'hybridome donnent une DO supérieure à 10 fois la DO du bruit de fond.

II) Western Blotting selon Burnette. Anal. Biochem. 1981, 112, 195-203.

Ce test permet de sélectionner les clones sécrétant des anticorps monoclonaux anti-villine de porc, reconnaissant également la villine humaine et la villine de poulet, parmi les clones positifs pour le test ELISA.

Différentes étapes du test :

a) Electrophorèse sur gel de polyacrylamide d'un extrait cellulaire de la muqueuse intestinale de poulet et d'un extrait cellulaire de la lignée HT29 (adénocarcinome de colon humain) exprimant la villine.

b) Electrotransfert des protéines séparées sur gel de polyacrylamide sur papier de nitrocellulose.

c) Incubation du papier de nitrocellulose sur lequel les protéines cellulaires ont été transférées avec les surnageants d'hybridomes préalablement sélectionnés en ELISA 1 nuit à 4°C.

d) Incubation 1 heure à température ambiante avec des anti-IgG de souris marquées à la peroxydase.

e) substrat :
- 10 mg tétrahydrochlorure de diaminobenzidine.
- 20 ml 0,1 M Tris HCl pH 7,6.
- 0,2 ml H$_2$O$_2$ à 1 %.

Tous les lavages entre chaque étape sont effectués avec du sérum de veau nouveau-né-PBS-Triton X100.

f) réaction positive : apparition rapide d'une bande marron (poids moléculaire 95Kd) si la réaction antigène-anticorps monoclonal a eu lieu.

D) CLONAGE DES HYBRIDES SELECTIONNES

- Méthode des dilutions limitées :
    Dilution en milieu sélectif des clones positifs de façon à ne distribuer qu'une cellule par puits (plaques 96 trous) sur des macrophages (origine souris Balb/C, 4 semaines) attachées au fond des puits.
    Addition de milieu sélectif conditionné.
- Sélection des clones positifs selon les méthodes précédemment énoncées.
- Reclonage si nécessaire.

E) PREPARATION D'ASCITES

- Les clones sélectionnés sont injectés à des souris (qui ont subi une injection de "PRISTANE" (Aldrich, 2,6,10,14-tétraméthylpentadécane) 4 à 5 jours avant).

- 1 à 2x10$^6$ cellules injectées/souris.
- Récupération au bout de 10 à 15 jours du liquide d'ascite contenant les cellules du clone qui ont proliféré. Le liquide d'ascite a un taux d'anticorps monoclonal anti-villine supérieur à 1 mg/ml.
  = Congélation des clones sélectionnés en milieu DMEM 10% FCS-5% DMSO.
    Conservation dans azote liquide à -176°C.

PROCEDE ELISA DIRECT POUR DOSER LA VILLINE.

= Adsorption des IgG purifiées à partir de l'ascite BD-D$_{2C3}$ en concentration constante sur des plaques ELISA.
  . L'anticorps a été purifié par chromatographie d'échange d'ions (DEAE-Tris acryl) et sur colonne d'hydroxyapatite.
  . Concentration à établir.
  . Incubation 2 heures à 37°C, puis 1 nuit à 4°C.
  . Lavage dans PBS/Tween 20 0,1 %.
= Saturation des plaques
    Incubation 30 minutes en présence de PBS/Tween 20 0,1%/BSA 0,4%.
= Addition de l'antigène
  . Gamme de concentration décroissante de la villine purifiée de 5 $\mu$g/ml à 1 ou 0,1 ng/ml en présence de BSA 0,4 %.
  . Incubation 3 heures à 4°C.
  . Lavages dans PBS/Tween 20 0,1 %.
= Addition des IgG purifiées à partir d'un sérum polyclonal de lapin dirigé contre la villine et couplées à la bêta-galactosidase.
  . Incubation 2 heures à 4°C.
  . Lavages dans PBS/Tween 20 0,1 %.
= Détection de la bêta-galactosidase
  . Addition de 20 mg de p-nitrophényl-bêta-D-galactopyrannoside à partir d'une solution à 4 mg/ml, dans 20 ml de tampon phosphate 0,1 M pH 7, MgSO$_4$ $10^{-3}$ M, MnSO$_4$ $2x10^{-4}$ M, EDTA (Merck 8409) $2x10^{-3}$ M.
  . Incubation x heures à 37°C.
  . Lecture densité optique à 414 nm.

EXEMPLE 3 : OBSERVATIONS EN CLINIQUE HUMAINE.

En utilisant un test ELISA décrit ci-après dont la sensibilité permet de détecter des taux très faibles de villine dans un extrait cellulaire (0,5 ng de villine par mg de protéines totales), on a effectué des mesures pour déceler des quantités dosables de villine dans le sérum. En effet, la villine étant une protéine intracellulaire, exprimée par les cellules normales et tumorales du tube digestif, les inventeurs ont vérifié que dans certaines conditions physiopathologiques, la nécrose des cellules contenant de la villine peut conduire à sa libération dans la circulation sanguine.

L'étude dans une population de donneurs de sang (n = 190) montre que la villine n'est pas détectable dans la très grande majorité des sérums (n = 168) ; cependant un petit nombre d'individus (n = 15) présente un taux de villine décelable (5 à 10 ng/ml). Cette valeur est retenue comme le niveau de villine n'ayant pas de signification pathologique. Enfin, quelques individus (n = 7) ont un niveau de villine supérieur à la valeur de base (50 à 100 ng/ml). Ces individus dit "normaux" (3,6%) n'ont pas été soumis a un examen clinique complémentaire de leurs voies digestives (fibroscopie, colonoscopie).

Les premiers résultats obtenus dans différentes pathologies gastro-intestinales sont résumes sur le tableau ci-après.

---

Détection de quantités de villine dosable (valeurs comprises entre 10 et 10.000 ng/ml) dans les sérums de malades porteurs de :

* <u>Pathologies malignes du tube digestif</u> :

- Cancers colorectaux.

- Cancers gastriques.

* <u>Pathologies bénignes du tube digestif</u> :

- Tumeurs villeuses.

- Maladies de Crohn.

- Recto-colite hemorragique.

- Ulcère du bulbe duodénal, gastrique, oesophagien.

---

Il est important de noter que par contre, les polyadénomes ("polypes") digestifs ne conduisent pas à la libération de cette protéine dans le sang.

Certains points complémentaires ont pu être déduits de cette étude :

1/ Il ne semble pas exister de corrélation entre la quantité villine circulante et la taille ou le stade évolutif de la tumeur.

2/ Lors du suivi post-opératoire, le dosage de cette protéine montre une chute de son taux sérique après exérèse complète. Par contre, en cas de persistance de tissu tumoral (exérèse incomplète, récidives, métastases), il est observé soit un niveau de villine maintenu, soit une augmentation de celui-ci.

Cette étude indique que la villine n'est pas habituellement détectable dans les sérums des sujets normaux, tandis que l'on peut la doser dans les sérums de malades atteints de pathologies malignes (cancers coliques ou du rectum) ou ayant des ulcérations bénignes (type maladies de Crohn, RCH, ulcère) du tube digestif faisant intervenir des processus inflammatoires et nécrotiques des muqueuses digestives.

Le dosage sanguin de la villine s'avère également d'un grand intérêt dans la surveillance des maladies bénignes (ulcérations et maladies inflammatoires des voies digestives). D'autre part, pour la recherche de villine circulante, il est possible d'utiliser, en association avec les anticorps de l'invention, d'autres marqueurs tumoraux comme l'ACE (antigène carcinogénique embryonnaire) qui est actuellement utilisé pour la surveillance évolutive des cancers mais dont les inconvénients principaux sont : 1/ d'être peu spécifique de l'organe atteint ; 2/ de s'observer dans des pathologies bénignes variées.

EXEMPLE -4: PREPARATION D'ADNc CORRESPONDANT A LA VILLINE HUMAINE.

Préparation des ARNm.

On prépare les ARNm à partir de la lignée cellulaire HT29 dérivée d'un adénocarcinome colique humain. Les ARN sont extraits par la méthode au chlorure de guanidium décrite par Ullrich et al. dans Science, 1977, 196, 1313-1319. La purification des ARNm polyA$^+$ est effectuée par chromatographie sur colonne d'oligo dT-cellulose selon la méthode décrite par Aviv et Leder dans Proc. Natl. Acad. Sci. USA, 1972, 69, 1408-1412.

Préparation des ADNc.

Le premier et second brin des ADNc sont préparés selon la méthode de Fiddes et Goodman décrite dans Nature, 1979, 281, 351-356. L'addition séquentielle des linkers Sall à l'extrémité 3' et Bam H1 à l'extrémité 5' est effectuée par la méthode d'Helfmann et al. rapportée dans Proc. Natl. Acad. Sci. USA, 1983, 80, 31-35.

On obtient une banque d'ADNc contenant environ 30 000 clones recombinants.

Vecteur.

On utilise des plasmides pEX1-3 (voir Stanley et Luzio, EMBO Journal, 1984, 3, 1429-1430, 1984). Ces vecteurs dérivent de plasmides contenant le gène de fusion cro-LacZ, dont l'expression est sous contrôle du promoteur PR du bactériophage lambda. Un polynucléotide contenant plusieurs sites uniques de restriction a été inséré à l'extrémité 3' du gène LacZ. Les signaux de terminaison de transcription et de traduction sont également insérés dans les trois phases de lecture.

Les ADNc ont été insérés dans chacun des plasmides pEX digérés par les enzymes de restriction Bam H1 SalI. Ils se trouvent donc tous dans la même orientation par rapport au gène CroLacZ.

Transformation.

On utilise comme souche bactérienne la souche E. coli pop 2135, construite par O. Raibaud comme décrit dans Nucleic Acid Research de la manière suivante :

On clone dans le site Bam HI d'un polylinker le fragment BglIII de 2,3 kb du phage lambda, portant l'allèle C1857 et le promoteur $P_R$ en opérant selon le schéma ci-dessous :

```
        BglII CI857 , P  BglII
                           R
            \              /
             \            /
              \          /
GAATTCGAGCTCGGTACCCGGGGATCCTCTAGAGTCGACCTGCAGGCATGCAAGCTTATCGAATTC

    EcoRI                      BamHI                              EcoRI
```

Le fragment EcoRI ainsi obtenu est alors cloné dans le site EcoRI de pOM41 et transféré dans le chromosome de la souche C600 (voir Gene, 29, 231-241).

Par cotransduction P1' avec un marqueur proximal aroB, on introduit cette structure dans le chromosome de MM294 ($F^-$ endA thi hsdR). On obtient E. coli pop 2135 : orientation malT, CI857, $P_R$, malPO.

La transformation de la souche bactérienne E. coli pop 2135 par les plasmides recombinants est réalisée selon la technique utilisant le rubidium décrite par Hanahan et al., J. Mol. Biol., 1983, 166, 557-580. Cette souche contient l'allèle clts857 du répresseur de cro.

Le nombre de recombinants obtenus est de l'ordre de $10^3$ à $10^4$ ng d'ADNc.

Sélection des clones par détection immunologique.

Les clones recombinants obtenus sont étalés sur filtres de nitrocellulose. La synthèse de la protéine hybride est induite après 2 heures d'incubation à 42°C. Après lyse des bactéries par le SDS et renaturation des protéines en l'absence de méthanol, les clones sont analysés par criblage immunologique. La renaturation des protéines est effectuée selon la technique de Burnett rapportée dans Anal. Biochem. 1981, 112, 195-203.

Dans un premier temps, la banque est criblée par un anticorps polyclonal dirigé contre la villine de porc intacte. On effectue ensuite un criblage secondaire en utilisant un anticorps polyclonal dirigé contre un fragment COOH-terminal de la villine de poulet et deux anticorps monoclonaux ($BDD_2C_3$ et $IID_3H_9$) reconnaissant les épitopes situés dans la région COOH-terminale de la molécule.

Le clone pEXZ-V19 contient un insérat de 510 paires de bases. La partie codante représente 330 paires de bases. Elle est suivie d'une région non codante de 200 paires de bases.

L'ADNc cloné code pour les 95 acides aminés COOH-terminaux de la villine humaine et représente environ le 1/10° de la protéine entière (poids moléculaire : 95 Kd).

REFERENCES

(1) Ullrich, A., Shine, J., Chirgwin, J., Pictet, R., Tischer, E., Rutter, W.J. & Goodman, H.M.
Rat insulin genes : construction of plasmids containing the coding sequences.
Science, 1977, 196, 1313-1319.

EP 0 206 849 B1

(2) Aviv, H. & Leder, P.

Purification of biologically active globin mRNA by chromatography on oligothymidylic acid cellulose. Proc. Natl. Acad. Sci. USA, 1972, 69, 1408-1412.

(3) Fiddes, J.C. & Goodman, H.M.

Isolation, cloning and sequence analysis of the cDNA for the alpha-subunit of human chorionic gonadotropin. Nature, 1979, 281, 351-355.

(4) Helfman, D.M., Feramisco, J.R., Fiddes, J.C., Thomas, G.P. & Hughes, S.H.

Identification of clones that encode chicken tropomyosin by direct immunological screening of a cDNA expression library.

Proc. Natl. Acad. Sci. USA, 1983, 80, 31-35.

(5) Stanley, K.K. & Luzio, J.P.

Construction of a new family of high efficiency bacterial expression vectors : identification of cDNA clones coding for human liver.

EMBO Journal, 1984, 3, 1429-1434.

(6) Stanley K.K.

Solubilization and immune-detection of beta-galactosidase hybrid proteins carrying foreign antigenic determinants.

Nucleic Acids Res., 1983, 11, 4077-4092.

(7) Hanahan, D.

Studies on transformation or E. coli with plasmids.

J. Mol. Biol., 1983, 166, 557-580.

(8) Burnett, W.N.

"Western Blotting" : Electrophoretic transfer of proteins from sodium dodecyl sulfate-polyacrylamide gels to unmodified nitrocellulose and radiographic detection with antibody and radioiodinated protein A.

Anal. Biochem., 1981, 112, 195-203.

**Revendications**

**Revendications pour les Etats contractants suivants : BE, CH, DE, FR, GB, IT, LI, LU, NL, SE**

1. Procédé de diagnostic pour la détection in vitro de cellules tumorales initialement formées dans la région digestive et/ou dérivées de celle-ci dans un prélèvement biologique suspecté d'avoir un caractère tumoral, caractérisé par la mise en contact de ce prélèvement biologique avec un réactif spécifique de la villine ou de son gène, constitué par des anticorps antivilline, ces anticorps reconnaissant la villine humaine, ou par un fragment d'ADN codant pour la villine humaine.

2. Anticorps monoclonal dirigé contre la villine caractérisé par les propriétés suivantes :
   - il reconnait la villine de porc intacte purifiée (Western Blotting),
   - il reconnait la villine d'extrait cellulaire de la lignée dérivée d'un adénocarcinome de colon humain (Western Blotting),
   - il reconnait la villine de rat en immunosytochimie (coupes congelées (cryostat) de muqueuse intestinale de rat fixées à la formaldéhyde),
   - il est produit par l'hybridome déposé le 29 Avril 1985 à la C.N.C.M. sous le n° I-440.

3. Anticorps monoclonal selon la revendication 2, caractérisé en ce qu'il reconnait un site antigénique contenu dans la séquence C-terminale (779-854) ou "partie de tête" de la villine de poulet de formule :

15

Val-Phe-Thr-Ala-Thr-Thr-Thr-Leu-Val-Pro-Thr-Lys-Leu-Glu-
Thr-Phe-Pro-Leu-Asp-Val-Leu-Val-Asn-Thr-Ala-Ala-Glu-Asp-
Leu-Pro-Arg-Gly-Val-Asp-Pro-Ser-Arg-Lys-Glu-Asn-His-Leu-
Ser-Asp-Glu-Asp-Phe-Lys-Ala-Val-Phe-Gly-Met-Thr-Arg-Ser-
Ala-Asn-Leu-Pro-Leu-Trp-Lys-Gln-Gln-Asn-Leu-Lys-Lys-Glu-

Glu-Lys-Gly-Leu-Phe

ledit anticorps monoclonal étant capable de reconnaitre ladite "partie de tête", que celle-ci soit isolée ou à l'intérieur du peptide 51 kd contenu dans la villine du poulet.

4.  Hybridome producteur de l'anticorps monoclonal selon la revendication 2, déposé à la C.N.C.M., sous le N° I-440 le 29 Avril 1985.

5.  Villine humaine biologiquement pure ne donnant essentiellement qu'une seule bande de poids moléculaire de 95 kD dans les essais de migration en électrophorèse sur gel de polyacrylamide.

6.  Procédé selon la revendication 1, caractérisé en ce que le réactif reconnaissant le gène codant pour la villine humaine est constitué par un ADN dont la séquence comporte une région codant pour une séquence d'acides aminés de la villine humaine, plus spécialement la partie COOH-terminale.

7.  Acide nucléique, caractérisé en ce qu'il correspond à l'ARNm complet de la villine humaine ou à un fragment d'ADN dont la séquence nucléotidique comporte une région codant pour une séquence d'acides aminés de la villine humaine, capable de s'hybrider spécifiquement avec une séquence nucléotidique codant pour la villine.

8.  Fragment d'ADNc, caractérisé par le fait qu'il renferme au moins une partie de la séquence nucléotidique codant pour les acides aminés de l'extrémité COOH de la villine humaine, l'enchaînement nucléotidique et celui des acides aminés correspondants présentant respectivement les séquences suivantes :

```
LYS TRP SER ASN THR LYS SER TYR GLU ASP LEU LYS ALA GLU
AAG TGG AGT AAC ACC AAA TCC TAT GAG GAC CTG AAG GCG GAG

SER GLY ASN SER ARG ASP TRP SER GLN ILE THR ALA GLU VAL
TCT GGC AAC TCT AGG GAC TGG AGC CAG ATC ACT GCT GAG GTC

THR SER PRO LYS VAL ASP VAL PHE ASN ALA ASN SER ASN LEU
ACA AGC CCC AAA GTG GAC GTG TTC AAT GCT AAC AGC AAC CTC

SER SER GLY PRO LEU PRO ILE PHE PRO LEU GLU GLN LEU VAL
AGT TCT GGG CCT CTG CCC ATC TTC CCC CTG GAG CAG CTA GTG

ASN LYS PRO VAL GLU GLU LEU PRO GLU GLY VAL ASP PRO SER
AAC AAG OCT GTA GAG GAG CTC CCC GAG GGT GTG GAC CCC AGC

ARG LYS GLU GLU HIS LEU SER ILE GLU ASP PHE THR GLN ALA
AGG AAG GAG GAA CAC CTG TCC ATT GAA GAT TTC ACT CAG GCC

PHE GLY MET THR PRO ALA ALA PHE SER ALA LEU PRO ARG TRP
TIT GGG ATG ACT CCA GCT GCC TTC TCT GCT CTG CCT CGA TGG

LYS GLN GLN ASN LEU LYS LYS GLU LYS GLY LEU PHE *
AAG CAA CAA AAC CTC AAG AAA GAA AAA GGA CTA TTT TGA GAAG
AGTAGCTGTGGTTGTAAAGCAGTACCCTACCCTGATTGTAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAAA
```

9. Sonde pour la détection des ARNm ou des ADN codant pour la villine humaine, caractérisée en ce qu'elle comprend au moins une partie de la séquence nucléotidique C-terminale selon la revendication 7, cette séquence étant le cas échéant marquée par un élément radioactif ou tout autre groupe permettant sa reconnaissance à l'état hybridé avec la préparation renfermant l'ARNm ou l'ADN à étudier.

10. Procédé d'obtention de la séquence nucléotidique codant pour l'extrémité C-terminale de la villine humaine, caractérisé en ce que :
- on prépare selon les techniques connues les ARNm (ARN messagers) totaux à partir d'une ligneé cellulaire exprimant la villine ;
- on construit une banque d'ADNc (ADN complémentaires) ;
- on insère les ADNc dans un vecteur susceptible d'exprimer la protéine codée par l'insérat ;
- on transforme à l'aide des vecteurs recombinants obtenus une souche bactérienne, puis on établit des conditions permettant l'expression de la protéine recherchée dans la bactérie ;
- on sélectionne les clones recombinants contenant le clone spécifique de la villine à l'aide d'anticorps reconnaissant la villine.

**11.** Procédé selon la revendication 10, caractérisé en ce que la construction de la banque d'ADNc est réalisée à l'aide d'un vecteur de clonage capable d'exprimer la protéine codée par l'ADNc inséré, plus spécialement par des plasmides de type pEX.

**12.** Procédé selon la revendication 10 ou 11, caractérisé en ce qu'on transforme une souche bactérienne du type E.coli, plus spécialement E.coli pop 2135, à l'aide des vecteurs recombinants renfermant une séquence nucléotidique codant pour l'extrémité C-Terminale de la villine humaine.

**13.** Procédé selon l'une quelconque des revendications 10 à 12, caractérisé en ce qu'on effectue un criblage de la banque d'ADNc à l'aide d'un anticorps anti-villine polyclonal tel qu'un anticorps polyclonal dirigé contre la villine de porc intacte puis un ou plusieurs anticorps monoclonaux reconnaissant des épitopes situés dans la région COOH-terminale de la molécule, le criblage à l'aide de l'anticorps polyclonal étant avantageusement suivi d'un criblage secondaire à l'aide d'un autre anticorps polyclonal, en particulier un anticorps polyclonal dirigé contre le fragment COOH-terminal de la villine de poulet.

**14.** Clone porteur d'un ADNc correspondant à la villine humaine, caractérisé en ce qu'il réagit spécifiquement avec l'anticorps polyclonal dirigé contre la villine intacte, avec l'anticorps polyclonal dirigé contre le peptide COOH-terminal de la villine de poulet et avec les deux anticorps monoclonaux dirigés contre des épitopes COOH-terminaux.

**Revendications pour l'Etat contractant suivant : AT**

**1.** Procédé de diagnostic pour la détection in vitro de cellules tumorales initialement formées dans la région digestive et/ou dérivées de celle-ci dans un prélèvement biologique suspecté d'avoir un caractère tumoral, caractérisé par la mise en contact de ce prélèvement biologique avec un réactif spécifique de la villine ou de son gène, constitué par des anticorps antivilline, ces anticorps reconnaissant la villine humaine, ou par un fragment d'ADN codant pour la villine humaine.

**2.** Procédé pour la préparation d'un anticorps monoclonal dirigé contre la villine comprenant
   - la fusion de cellules spléniques d'une souris préalablement immunisée avec la villine de porc, avec des cellules de myélome,
   - la sélection des hybridomes secréteurs d'un anticorps monoclonal ayant les propriétés suivantes :
   - il reconnait la villine de porc intacte purifiée (Western Blotting),
   - il reconnait la villine d'extrait cellulaire de la lignée dérivée d'un adénocarcinome de colon humain (Western Blotting),
   - il reconnait la villine de rat en immunocytochimie (coupes congelées (cryostat) de muqueuse intestinale de rat fixées à la formaldéhyde),
   - il est produit par l'hybridome déposé le 29 Avril 1985 à la C.N.C.M. sous le n° I-440.

**3.** Procédé pour la préparation d'un anticorps monoclonal selon la revendication 2, caractérisé en ce que l'anticorps sélectionné reconnait un site antigénique contenu dans la séquence C-terminale (779-854) ou "partie de tête" de la villine de poulet de formule :

```
            1                                              10   11
Val-Phe-Thr-Ala-Thr-Thr-Thr-Leu-Val-Pro-Thr-Lys-Leu-Glu-
                               20
Thr-Phe-Pro-Leu-Asp-Val-Leu-Val-Asn-Thr-Ala-Ala-Glu-Asp-
        30   31                                        40   41
Leu-Pro-Arg-Gly-Val-Asp-Pro-Ser-Arg-Lys-Glu-Asn-His-Leu-
                             50   51
Ser-Asp-Glu-Asp-Phe-Lys-Ala-Val-Phe-Gly-Met-Thr-Arg-Ser-
        60   61                                        70   71
Ala-Asn-Leu-Pro-Leu-Trp-Lys-Gln-Gln-Asn-Leu-Lys-Lys-Glu-


Glu-Lys-Gly-Leu-Phe
```

ledit anticorps monoclonal étant capable de reconnaitre ladite "partie de tête", que celle-ci soit isolée ou à l'intérieur du peptide 51 kd contenu dans la villine du poulet.

4. Procédé pour la préparation d'un hybridome producteur de l'anticorps monoclonal produit selon la revendication 2, comprenant la fusion de cellules spléniques d'une souris préalablement immunisée avec la villine de porc, avec des cellules de myélome et la récupération de l'hybridome déposé à la C.N.C.M., sous le N° I-440 le 29 Avril 1985.

5. Procédé de préparation de la villine humaine biologiquement pure caractérisé en ce qu'il comprend:
   - le traitement d'un lysat d'entérocytes humains avec une solution aqueuse contenant un détergent approprié et la récupération d'une solution contenant la villine humaine,
   - le passage de la solution contenant la villine humaine au contact d'une colonne d'affinité portant un anticorps monoclonal tel qu'obtenu selon la revendication 2 ou 3, dans des conditions permettant la fixation sélective de la villine humaine par l'anticorps,
   - la récupération de la villine humaine par dissociation du complexe antigène-anticorps, ladite villine ne donnant essentiellement qu'une seule bande de poids moléculaire de 95 kD dans les essais de migration en électrophorèse sur gel de polyacrylamide.

6. Procédé selon la revendication 1, caractérisé en ce que le réactif reconnaissant le gène codant pour la villine humaine est constitué par un ADN dont la séquence comporte une région codant pour une séquence d'acides aminés de la villine humaine, plus spécialement la partie COOH-terminale.

7. Procédé de préparation d'acide nucléique correspondant à l'ARNm complet de la villine humaine ou à un fragment d'ADN dont la séquence nucléotidique comporte une région codant pour une séquence d'acides aminés de la villine humaine, capable de s'hybrider spécifiquement avec une séquence nucléotidique codant pour la villine, caractérisé en qu'il comprend:
   - l'extraction de l'ARN à partir d'une lignée cellulaire exprimant la villine humaine,
   - la récupération de l'ARNm et le cas échéant,
   - l'obtention d'ADN à partir de cet ARNm.

8. Procédé de préparation d'un fragment d'ADNc, caractérisé par
   - la construction d'une banque d'ADNc,
   - la sélection d'un fragment d'ADNc renfermant au moins une partie de la séquence nucléotidique codant pour les acides aminés de l'extrémité COOH de la villine humaine, l'enchaînement nucléotidique et celui des acides aminés correspondants présentant respectivement les séquences suivantes :

```
LYS TRP SER ASN THR LYS SER TYR GLU ASP LEU LYS ALA GLU
AAG TGG AGT AAC ACC AAA TCC TAT GAG GAC CTG AAG GCG GAG

SER GLY ASN SER ARG ASP TRP SER GLN ILE THR ALA GLU VAL
TCT GGC AAC TCT AGG GAC TGG AGC CAG ATC ACT GCT GAG GTC

THR SER PRO LYS VAL ASP VAL PHE ASN ALA ASN SER ASN LEU
ACA AGC CCC AAA GTG GAC GTG TTC AAT GCT AAC AGC AAC CTC

SER SER GLY PRO LEU PRO ILE PHE PRO LEU GLU GLN LEU VAL
AGT TCT GGG CCT CTG CCC ATC TTC CCC CTG GAG CAG CTA GTG

ASN LYS PRO VAL GLU GLU LEU PRO GLU GLY VAL ASP PRO SER
AAC AAG OCT GTA GAG GAG CTC CCC GAG GGT GTG GAC CCC AGC

ARG LYS GLU GLU HIS LEU SER ILE GLU ASP PHE THR GLN ALA
AGG AAG GAG GAA CAC CTG TCC ATT GAA GAT TTC ACT CAG GCC

PHE GLY MET THR PRO ALA ALA PHE SER ALA LEU PRO ARG TRP
TIT GGG ATG ACT CCA GCT GCC TTC TCT GCT CTG CCT CGA TGG

LYS GLN GLN ASN LEU LYS LYS GLU LYS GLY LEU PHE *
AAG CAA CAA AAC CTC AAG AAA GAA AAA GGA CTA TTT TGA GAAG
AGTAGCTGTGGTTGTAAAGCAGTACCCTACCCTGATTGTAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAA
```

9. Procédé pour la préparation d'une sonde pour la détection des ARNm ou des ADN codant pour la villine humaine, comprenant au moins une partie de la séquence nucléotidique C-terminale telle qu'obtenue par un procédé selon la revendication 7, comprenant le marquage de cette séquence par un élément radioactif ou tout autre groupe permettant sa reconnaissance à l'état hybridé avec la préparation renfermant l'ARNm ou l'ADN à étudier.

10. Procédé d'obtention de la séquence nucléotidique codant pour l'extrémité C-Terminale de la villine humaine, caractérisé en ce que :
- on prépare selon les techniques connues les ARNm (ARN messagers) totaux à partir d'une lignée cellulaire exprimant la villine ;
- on construit une banque d'ADNc (ADN complémentaires) ;
- on insère les ADNc dans un vecteur susceptible d'exprimer la protéine codée par l'insérat ;
- on transforme à l'aide des vecteurs recombinants obtenus une souche bactérienne, puis on établit des conditions permettant l'expression de la protéine recherchée dans la bactérie ;
- on sélectionne les clones recombinants contenant le clone spécifique de la villine à l'aide d'anticorps reconnaissant la villine.

**11.** Procédé selon la revendication 10, caractérisé en ce que la construction de la banque d'ADNc est réalisée à l'aide d'un vecteur de clonage capable d'exprimer la protéine codée par l'ADNc inséré, plus spécialement par des plasmides de type pEX.

**12.** Procédé selon la revendication 10 ou 11, caractérisé en ce qu'on transforme une souche bactérienne du type E.coli, plus spécialement E.coli pop 2135, à l'aide des vecteurs recombinants renfermant une séquence nucléotidique codant pour l'extrémité C-Terminale de la villine humaine.

**13.** Procédé selon l'une quelconque des revendications 10 à 12, caractérisé en ce qu'on effectue un criblage de la banque d'ADNc à l'aide d'un anticorps anti-villine polyclonal tel qu'un anticorps polyclonal dirigé contre la villine de porc intacte puis un ou plusieurs anticorps monoclonaux reconnaissant des épitopes situés dans la région COOH-terminale de la molécule, le criblage à l'aide de l'anticorps polyclonal étant avantageusement suivi d'un criblage secondaire à l'aide d'un autre anticorps polyclonal, en particulier un anticorps polyclonal dirigé contre le fragment COOH-Terminal de la villine de poulet.

**14.** Préparation d'un clone porteur d'un ADNc correspondant à la villine humaine, caractérisé en ce qu'à l'issue du procédé selon la revendication 10 on sélectionne un clone qui réagit spécifiquement avec l'anticorps polyclonal dirigé contre la villine intacte, avec l'anticorps polyclonal dirigé contre le peptide COOH-terminal de la villine de poulet et avec les deux anticorps monoclonaux dirigés contre des épitopes COOH-terminaux.

**Claims**
**Claims for the following Contracting States : BE, CH, DE, FR, GB, IT, LI, LU, NL, SE**

**1.** Diagnostic procedure for the in vitro detection of tumor cells formed initially in the digestive region and/or derived from these latter in a biological sample suspected to have tumoral character, characterized by placing this biological sample in contact with a specific reagent for villin or its gene, constituted by anti-villin antibodies, these antibodies recognizing human villin, or by a DNA fragment coding for human villin.

**2.** Monoclonal antibody directed against villin characterized by the following properties :
- it recognizes purified intact porcine villin (Western blotting)
- it recognizes the villin of a cell extract of the line derived from a human colon adenocarcinoma (Western blotting)-
- it recognizes rat villin in immunocytochemistry (frozen sections (cryostat) of rat intestinal mucosa fixed with formaldehyde),
- it is produced by the hybridoma deposited with the CNCM on 29 April 1985 under the No. I-440.

**3.** Monoclonal antibody according to Claim 2, characterized in that it recognizes an antigenic site contained in the C-terminal sequence (779-854) or the "head part" of chicken villin of formula :

```
    1                                           10    11
Val-Phe-Thr-Ala-Thr-Thr-Thr-Leu-Val-Pro-Thr-Lys-Leu-Glu-
                            20
Thr-Phe-Pro-Leu-Asp-Val-Leu-Val-Asn-Thr-Ala-Ala-Glu-Asp-
        30   31                                    40    41
Leu-Pro-Arg-Gly-Val-Asp-Pro-Ser-Arg-Lys-Glu-Asn-His-Leu-
                            50   51
Ser-Asp-Glu-Asp-Phe-Lys-Ala-Val-Phe-Gly-Met-Thr-Arg-Ser-
        60   61                                    70    71
Ala-Asn-Leu-Pro-Leu-Trp-Lys-Gln-Gln-Asn-Leu-Lys-Lys-Glu-

Glu-Lys-Gly-Leu-Phe
```

the said monoclonal antibody being capable of recognizing the said "head part" irrespective of whether it exists in isolation or forms an integral part of the 51 kD peptide contained in chicken villin.

4. Hybridoma producing the monoclonal antibody according to Claim 2 deposited with the C.N.C.M. under the No. I-440 on 29 April 1985.

5. Biologically pure human villin giving essentially only a single band of 95 kD molecular weight when allowed to migrate on polyacrylamide gel electrophoresis.

6. Procedure according to Claim 1, characterized in that the reagent recognizing the gene coding for human villin is constituted by a DNA, the sequence of which comprises a region coding for an amino acid sequence of human villin, more particularly the COOH-terminal part.

7. Nucleic acid, characterized in that it corresponds to the complete mRNA of human villin or to a DNA fragment, the nucleotide sequence of which comprises a region coding for an amino acid sequence of human villin capable of hybridizing specifically with a nucleotide sequence coding for villin.

8. cDNA fragment characterized by the fact that it contains at least a part of the nucleotide sequence coding for the amino acids of the COOH terminus of human villin, the nucleotide sequence and that of the corresponding amino acids possessing respectively the following sequences :

```
LYS TRP SER ASN THR LYS SER TYR GLU ASP LEU LYS ALA GLU
AAG TGG AGT AAC ACC AAA TCC TAT GAG GAC CTG AAG GCG GAG

SER GLY ASN SER ARG ASP TRP SER GLN ILE THR ALA GLU VAL
TCT GGC AAC TCT AGG GAC TGG AGC CAG ATC ACT GCT GAG GTC

THR SER PRO LYS VAL ASP VAL PHE ASN ALA ASN SER ASN LEU
ACA AGC CCC AAA GTG GAC GTG TTC AAT GCT AAC AGC AAC CTC

SER SER GLY PRO LEU PRO ILE PHE PRO LEU GLU GLN LEU VAL
AGT TCT GGG CCT CTG CCC ATC TTC CCC CTG GAG CAG CTA GTG

ASN LYS PRO VAL GLU GLU LEU PRO GLU GLY VAL ASP PRO SER
AAC AAG OCT GTA GAG GAG CTC CCC GAG GGT GTG GAC CCC AGC

ARG LYS GLU GLU HIS LEU SER ILE GLU ASP PHE THR GLN ALA
AGG AAG GAG GAA CAC CTG TCC ATT GAA GAT TTC ACT CAG GCC

PHE GLY MET THR PRO ALA ALA PHE SER ALA LEU PRO ARG TRP
TIT GGG ATG ACT CCA GCT GCC TTC TCT GCT CTG CCT CGA TGG

LYS GLN GLN ASN LEU LYS LYS GLU LYS GLY LEU PHE *
AAG CAA CAA AAC CTC AAG AAA GAA AAA GGA CTA TTT TGA GAAG
AGTAGCTGTGGTTGTAAAGCAGTACCCTACCCTGATTGTAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAAA
```

9. Probe for the detection of the mRNAs or the DNAs coding for human villin, characterized in that it comprises at least a part of the C-terminal nucleotide sequence according to Claim 7, this sequence being labelled if necessary by a radioactive element or any other group making possible its recognition in the hybridized state with the preparation containing the mRNA or the DNA to be studied.

10. Procedure for obtaining the nucleotide sequence coding for the C-terminus of human villin, characterized in that :
   - the total mRNAs (messenger RNAs) are prepared from a cell line expressing villin according to known procedures;
   - a cDNA (complementary DNA) library is constructed ;
   - the cDNAs are inserted into a vector capable of expressing the protein encoded in the insert;
   - a bacterial strain is transformed with the aid of the recombinant vectors obtained, then conditions are defined which allow the expression of the desired protein in the bacteria;
   - the recombinant clones containing the clone specific for villin is selected with the aid of antibodies recognizing villin.

11. Procedure according to Claim 10, characterized in that the construction of the cDNA library is achieved with the aid of a cloning vector capable of expressing the protein encoded in the inserted cDNA, more especially by plasmids of the pEX type.

12. Procedure according to Claim 10 or 11, characterized in that a bacterial strain of the E. coli type, more particularly E. coli pop 2135, is transformed with the aid of the recombinant vectors containing a nucleotide sequence coding for the C-terminus of human villin.

13. Procedure according to any one of the Claims 10 to 12, characterized in that a screening of the cDNA library is carried out using a polyclonal anti-villin antibody such as a polyclonal antibody directed against intact porcine villin, then one or more monoclonal antibodies which recognize epitopes located in the COOH-terminal region of the molecule, the screening with the aid of the polyclonal antibody being advantageously followed by a secondary screening using another polyclonal antibody, in particular a polyclonal antibody directed against the COOH-terminal fragment of chicken villin.

14. Clone bearing a cDNA corresponding to human villin, characterized in that it reacts specifically with the polyclonal antibody directed against intact villin, with the polyclonal antibody directed against the COOH-terminal peptide of chicken villin and with the two monoclonal antibodies directed against COOH-terminal epitopes.

**Claims for the following Contracting State : AT**

1. Diagnostic procedure for the in vitro detection of tumor cells formed initially in the digestive region and/or derived from these latter in a biological sample suspected to have tumoral character, characterized by placing this biological sample in contact with a specific reagent for villin or its gene, constituted by anti-villin antibodies, these antibodies recognizing human villin, or by a DNA fragment coding for human villin.

2. Procedure for the preparation of a monoclonal antibody directed against villin comprising :
   - the fusion of spleen cells of a mouse immunized beforehand with porcine villin with myeloma cells,
   - the selection of hybridomas secreting a monoclonal antibody which has the following properties :
   - it recognizes purified intact porcine villin (Western blotting)
   - it recognizes the villin of a cell extract of the line derived from a human colon adenocarcinoma (Western blotting)-
   - it recognizes rat villin in immunocytochemistry (frozen sections (cryostat) of rat intestinal mucosa fixed with formaldehyde),
   - it is produced by the hybridoma deposited with the CNCM on 29 April 1985 under the No. I-440.

3. Procedure for the preparation of a monoclonal antibody according to Claim 2, characterized in that the antibody selected recognizes an antigenic site contained in the C-terminal sequence (779-854) or the "head part" of chicken villin of formula :

```
         1                                                10   11
Val-Phe-Thr-Ala-Thr-Thr-Thr-Leu-Val-Pro-Thr-Lys-Leu-Glu-
                               20
Thr-Phe-Pro-Leu-Asp-Val-Leu-Val-Asn-Thr-Ala-Ala-Glu-Asp-
           30   31                                    40   41
Leu-Pro-Arg-Gly-Val-Asp-Pro-Ser-Arg-Lys-Glu-Asn-His-Leu-
                          50   51
Ser-Asp-Glu-Asp-Phe-Lys-Ala-Val-Phe-Gly-Met-Thr-Arg-Ser-
           60   61                                    70   71
Ala-Asn-Leu-Pro-Leu-Trp-Lys-Gln-Gln-Asn-Leu-Lys-Lys-Glu-

Glu-Lys-Gly-Leu-Phe
```

the said monoclonal antibody being capable of recognizing the said "head part" irrespective of whether it exists in isolation or forms an integral part of the 51 kD peptide contained in chicken villin.

4. Procedure for the preparation of a hybridoma producing the monoclonal antibody obtained according to Claim 2, comprising the fusion of spleen cells of a mouse immunized beforehand with porcine villin with myeloma cells, and the recovery of the hybridoma deposited with the C.N.C.M. under the No. I-440 on 29 April 1985.

5. Procedure for the preparation of biologically pure human villin characterized in that it comprises :
   - the treatment of a lysate of human enterocytes with an aqueous solution containing a suitable detergent and the recovery of a solution containing human villin,
   - the passage of the solution containing human villin through an affinity column bearing a monoclonal antibody such as that obtained according to Claim 2 or 3 under conditions allowing the selective binding of the human villin by the antibody,
   - the recovery of the human villin by dissociation of the antigen-antibody complex, the said villin giving essentially only a single band of 95 kD molecular weight when allowed to migrate on polyacrylamide gel electrophoresis.

6. Procedure according to Claim 1, characterized in that the reagent recognizing the gene coding for human villin is constituted by a DNA, the sequence of which comprises a region coding for an amino acid sequence of human villin, more particularly the COOH-terminal part.

7. Procedure for the preparation of a nucleic acid corresponding to the complete mRNA of human villin or to a DNA fragment, the nucleotide sequence of which comprises a region coding for an amino acid sequence of human villin capable of hybridizing specifically with a nucleotide sequence coding for villin, characterized in that it comprises :
   - the extraction of the RNA from a cell line expressing human villin,
   - the recovery of the mRNA and, if necessary,
   - the production of DNA from this mRNA.

8. Procedure for the preparation of a cDNA fragment, characterized by
   - the construction of a cDNA library,
   - the selection of a cDNA fragment containing at least a part of the nucleotide sequence coding for the amino acids of the COOH terminus of human villin, the nucleotide sequence and that of the corresponding amino acids possessing respectively the following sequences :

```
LYS TRP SER ASN THR LYS SER TYR GLU ASP LEU LYS ALA GLU
AAG TGG AGT AAC ACC AAA TCC TAT GAG GAC CTG AAG GCG GAG

SER GLY ASN SER ARG ASP TRP SER GLN ILE THR ALA GLU VAL
TCT GGC AAC TCT AGG GAC TGG AGC CAG ATC ACT GCT GAG GTC

THR SER PRO LYS VAL ASP VAL PHE ASN ALA ASN SER ASN LEU
ACA AGC CCC AAA GTG GAC GTG TTC AAT GCT AAC AGC AAC CTC

SER SER GLY PRO LEU PRO ILE PHE PRO LEU GLU GLN LEU VAL
AGT TCT GGG CCT CTG CCC ATC TTC CCC CTG GAG CAG CTA GTG

ASN LYS PRO VAL GLU GLU LEU PRO GLU GLY VAL ASP PRO SER
AAC AAG OCT GTA GAG GAG CTC CCC GAG GGT GTG GAC CCC AGC

ARG LYS GLU GLU HIS LEU SER ILE GLU ASP PHE THR GLN ALA
AGG AAG GAG GAA CAC CTG TCC ATT GAA GAT TTC ACT CAG GCC

PHE GLY MET THR PRO ALA ALA PHE SER ALA LEU PRO ARG TRP
TIT GGG ATG ACT CCA GCT GCC TTC TCT GCT CTG CCT CGA TGG

LYS GLN GLN ASN LEU LYS LYS GLU LYS GLY LEU PHE *
AAG CAA CAA AAC CTC AAG AAA GAA AAA GGA CTA TTT TGA GAAG
AGTAGCTGTGGTTGTAAAGCAGTACCCTACCCTGATTGTAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAAA
```

9. Procedure for the preparation of a probe for the detection of the mRNAs or the DNAs coding for human villin, comprising at least a part of the C-terminal nucleotide sequence such as that obtained by a procedure according to Claim 7, including the labelling of this sequence by a radioactive element or any other group making possible its recognition in the hybridized state with the preparation containing the mRNA or the DNA to be studied.

10. Procedure for obtaining the nucleotide sequence coding for the C-terminus of human villin, characterized in that :
- the total mRNAs (messenger RNAs) are prepared from a cell line expressing villin according to known procedures;
- a cDNA (complementary DNA) library is constructed ;
- the cDNAs are inserted into a vector capable of expressing the protein encoded in the insert;
- a bacterial strain is transformed with the aid of the recombinant vectors obtained, then conditions are defined which allow the expression of the desired protein in the bacteria;
- the recombinant clones containing the clone specific for villin is selected with the aid of antibodies recognizing villin.

11. Procedure according to Claim 10, characterized in that the construction of the cDNA library is achieved with the aid of a cloning vector capable of expressing the protein encoded in the inserted cDNA, more especially by plasmids of the pEX type.

12. Procedure according to Claim 10 or 11, characterized in that a bacterial strain of the E. coli type, more particularly E. coli pop 2135, is transformed with the aid of the recombinant vectors containing a nucleotide sequence coding for the C-terminus of human villin.

13. Procedure according to any one of the Claims 10 to 12, characterized in that a screening of the cDNA library is carried out using a polyclonal anti-villin antibody such as a polyclonal antibody directed against intact porcine villin, then one or more monoclonal antibodies which recognize epitopes located in the COOH-terminal region of the molecule, the screening with the aid of the polyclonal antibody being advantageously followed by a secondary screening using another polyclonal antibody, in particular a polyclonal antibody directed against the COOH-terminal fragment of chicken villin.

14. Preparation of a clone bearing a cDNA corresponding to human villin, characterized in that on completion of the procedure according to Claim 10 a clone is selected which reacts specifically with the polyclonal antibody directed against intact villin, with the polyclonal antibody directed against the COOH-terminal peptide of chicken villin and with the two monoclonal antibodies directed against COOH-terminal epitopes.

**Patentansprüche**
**Patentansprüche für folgende Vertragsstaaten : BE, CH, DE, FR, GB, IT, LI, LU, NL, SE**

1. Diagnoseverfahren zum in vitro-Nachweis von Tumorzellen, die ursprünglich im Verdauungstrakt gebildet worden sind, und/oder Derivaten davon in einer biologischen Probe, von der angenommen wird, daß sie Tumorcharakter besitzt, **dadurch gekennzeichnet,** daß man diese biologische Probe mit einem für Villin oder sein Gen spezifischen Reagens in Kontakt bringt, welches aus Antivillin-Antikörpern, diese Antikörper, die Human-Villin erkennen, oder aus einem DNA-Fragment, welches für Human-Villin codiert, gebildet ist.

2. Gegen Villin gerichteter monoklonaler Antikörper, **gekennzeichnet durch** die folgenden Eigenschaften:
   - er erkennt gereinigtes intaktes Schweine-Villin (Western Blotting),
   - er erkennt das Villin des Zellextrakts der Zellinie, die von einem Adenokarzinom des menschlichen Colons abgeleitet ist (Western Blotting),
   - er erkennt Ratten-Villin in der Immunocytochemie (mit Formaldehyd fixierte Gefrierschnitte (Kryostat) von Ratten-Darmschleimhaut),
   - er wird durch das am 29. April 1985 unter der Hinterlegungsnummer I-440 beim C.N.C.M. hinterlegte Hybridom produziert.

3. Monoklonaler Antikörper nach Anspruch 2, **dadurch gekennzeichnet,** daß er eine antigene Stelle in der C-terminalen Sequenz (779-854) oder dem "Kopfteil" des Hühner-Villins der Formel erkennt:

1              10 11

Val-Phe-Thr-Ala-Thr-Thr-Thr-Leu-Val-Pro-Thr-Lys-Leu-Glu-Thr-Phe-Pro-

     20             30 31

Leu-Asp-Val-Leu-Val-Asn-Thr-Ala-Ala-Glu-Asp-Leu-Pro-Arg-Gly-Val-Asp-

        40 41            50 51

Pro-Ser-Arg-Lys-Glu-Asn-His-Leu-Ser-Asp-Glu-Asp-Phe-Lys-Ala-Val-Phe-

            60 61           70

Gly-Met-Thr-Arg-Ser-Ala-Asn-Leu-Pro-Leu-Trp-Lys-Gln-Gln-Asn-Leu-Lys-

71

Lys-Glu-Glu-Lys-Gly-Leu-Phe

wobei dieser monoklonale Antikörper in der Lage ist, diesen "Kopfteil" zu erkennen, ob dieser isoliert oder im Inneren des im Hühner-Villin vorliegenden Peptids mit 51 kd vorhanden ist.

**4.** Hybridom zur Bildung des monoklonalen Antikörpers gemäß Anspruch 2, hinterlegt am 29. April 1985 unter der Hinterlegungsnummer I-440 am C.N.C.M.

**5.** Biologisch reines Human-Villin, welches bei der Untersuchung der elektrophoretischen Wanderung auf Polyacrylamidgel im wesentlichen nur eine Bande des Molekulargewichts 95 kD ergibt.

**6.** Verfahren nach Anspruch 1**, dadurch gekennzeichnet,** daß das Reagens, welches das Human-Villin codierende Gen erkennt, aus einer DNA gebildet ist, deren Sequenz einen Bereich aufweist, der für eine Aminosäuresequenz des Human-Villins codiert, insbesondere den COOH-terminalen Bereich.

**7.** Nucleinsäure, **dadurch gekennzeichnet,** daß sie der vollständigen mRNA des Human-Villins oder einem DNA-Fragment entspricht, dessen Nucleotidsequenz einen Bereich aufweist, der für eine Aminosäuresequenz des Human-Villins codiert, der dazu in der Lage ist, sich spezifisch mit einer für Villin codierenden Nucleotidsequenz zu hybridisieren.

**8.** cDNA-Fragment, **dadurch gekennzeichnet,** daß es mindestens einen Teil der Nucleotidsequenz enthält, die für Aminosäuren des COOH-Endes des Human-Villins codiert, wobei die Nucleotidkette und die Kette der entsprechenden Aminosäuren die folgenden Sequenzen aufweisen:

LYS TRP SER ASN THR LYS SER TYR GLU ASP LEU LYS ALA GLU
AAG TGG AGT AAC ACC AAA TCC TAT GAG GAC CTG AAG GCG GAG

SER GLY ASN SER ARG ASP TRP SER GLN ILE THR ALA GLU VAL
TCT GGC AAC TCT AGG GAC TGG AGC CAG ATC ACT GCT GAG GTC

THR SER PRO LYS VAL ASP VAL PHE ASN ALA ASN SER ASN LEU
ACA AGC CCC AAA GTG GAC GTG TTC AAT GCT AAC AGC AAC CTC

SER  SER  GLY  PRO LEU  PRO  ILE   PHE  PRO  LEU   GLU  GLN LEU VAL

AGT  TCT  GGG  CCT  CTG  CCC ATC  TTC   CCC  CTG  GAG  CAG CTA GTG


ASN  LYS  PRO  VAL  GLU  GLU LEU  PRO  GLU  GLY  VAL  ASP  PRO SER

AAC  AAG  CCT  GTA  GAG  GAG CTC  CCC  GAG  GGT  GTG GAC  CCC AGC


ARG  LYS  GLU   GLU  HIS  LEU SER  ILE   GLU  ASP  PHE  THR GLN  ALA

AGG  AAG  GAG  GAA  CAC CTG TCC  ATT  GAA  GAT  TTC  ACT  CAG  GCC


PHE  GLY  MET  THR  PRO  ALA  ALA   PHE  SER  ALA  LEU  PRO  ARG  TRP

TIT   GGG ATG   ACT  CCA  GCT  GCC TTC   TCT  GCT  CTG  CCT  CGA  TGG


LYS GLN GLN ASN LEU LYS LYS GLU LYS GLY LEU PHE *

AAG CAA CAA AAC CTC AAG AAA GAA AAA GGA CTA TTT TGA GAAG

AGTAGCTGTGGTTGTAAAGCAGTACCCTACCCTGATTGTAGGGTCTCATTTTCTCA

CCGATATTAGTCCTACACCAATTGAAGTGAAATTTTGCAGATGTGCCTATGAGCAC

AAACTTCTGTGGCAAATGCCAGTTTTGTTTAATAAATGTACCTATTCCTTCAGAAA

GATGATACCCCAAAAAAAAAAAA


**9.** Sonde zum Nachweis von mRNA oder DNA, die für Human-Villin codieren, **dadurch gekennzeichnet,** daß sie mindestens einen Teil der C-terminalen Nucleotidsequenz gemäß Anspruch 7 umfaßt, wobei diese Sequenz gegebenenfalls durch ein radioaktives Element oder irgendeine andere Gruppe, die ihre Erkennung in hybridisiertem Zustand mit dem Präparat, das die zu untersuchende mRNA oder DNA enthält, ermöglicht.

**10.** Verfahren zur Herstellung der für das C-terminale Ende des Human-Villins codierenden Nucleotidsequenz, **dadurch gekennzeichnet,** daß man
- mit Hilfe an sich bekannter Verfahrensweisen ausgehend von einer Zellinie, die Villin exprimiert, vollständige mRNA (Messenger-RNA) herstellt;
- eine cDNA-Bank bildet (komplementäre DNA);
- die cDNA in einen Vektor einfügt, der in der Lage ist, das durch die Einfügung codierte Protein zu exprimieren;
- mit Hilfe der erhaltenen rekombinanten Vektoren einen Bakterienstamm transformiert und dann Bedingungen einstellt, die die Expression des gewünschten Proteins in dem Bakterium ermöglichen;
- mit Hilfe von Villin erkennenden Antikörpern die rekombinanten Klone selektioniert, welche den für Villin spezifischen Klon enthalten.

**11.** Verfahren nach Anspruch 10, **dadurch gekennzeichnet,** daß der Aufbau der cDNA-Bank mit Hilfe eines Klonierungsvektors bewirkt wird, der dazu geeignet ist, das durch die eingefügte cDNA codierte Protein zu exprimieren, insbesondere mit Hilfe von Plasmiden des Typs pEX.

**12.** Verfahren nach Anspruch 10 oder 11, **dadurch gekennzeichnet,** daß man einen Bakterienstamm des Typs E. coli, insbesondere E. coli pop 2135, mit Hilfe von rekombinanten Vektoren, welche eine für das C-terminale Ende des Human-Villins codierende Nucleotidsequenz enthalten, transformiert.

**13.** Verfahren nach einem der Ansprüche 10 bis 12, **dadurch gekennzeichnet,** daß man ein Screening der cDNA-Bank mit Hilfe eines polyklonalen Anti-Villin-Antikörpers, wie eines gegen intaktes Schweine-Villin gerichteten polyklonalen Antikörpers, und dann mit einem oder mehreren monoklonalen Antikörpern, die in dem COOH-terminalen Bereich vorliegende Epitope erkennen, bewirkt, wobei das Screening mit Hilfe der polyklonalen Antikörper mit Vorteil von einem sekundären Screening mit Hilfe eines weiteren polyklonalen Antikörpers gefolgt wird, insbesondere mit Hilfe eines gegen das COOH-terminale Fragment des Hühner-Villins gerichteten polyklonalen Antikörpers.

**14.** Klon, der eine dem Human-Villin entsprechende cDNA trägt**, dadurch gekennzeichnet,** daß er spezifisch mit dem gegen das intakte Villin gerichteten polyklonalen Antikörper, mit dem gegen das COOH-terminale Peptid des Hühner-Villins gerichteten polyklonalen Antikörper und mit den beiden, gegen die COOH-terminalen Epitope gerichteten monoklonalen Antikörper reagiert.

**Patentansprüche für folgenden Vertragsstaat : AT**

**1.** Diagnoseverfahren zum in vitro-Nachweis von Tumorzellen, die ursprünglich im Verdauungstrakt gebildet worden sind, und/oder Derivaten davon in einer biologischen Probe, von der angenommen wird, daß sie Tumorcharakter besitzt, **dadurch gekennzeichnet,** daß man diese biologische Probe mit einem für Villin oder sein Gen spezifischen Reagens in Kontakt bringt, welches aus Antivillin-Antikörpern, diese Antikörper, die Human-Villin erkennen, oder aus einem DNA-Fragment, welches für Human-Villin codiert, gebildet ist.

**2.** Verfahren zur Herstellung eines gegen Villin gerichteten monoklonalen Antikörpers, umfassend:
 - die Fusion von Milzzellen einer Maus, die zuvor mit Schweine-Villin immunisiert worden ist, mit Myelomzellen,
 - die Selektion von Hybridomen, die einen monoklonalen Antikörper bilden, der die folgenden Eigenschaften besitzt:
 - er erkennt gereinigtes intaktes Schweine-Villin (Western Blotting),
 - er erkennt das Villin des Zellextrakts der Zellinie, die von einem Adenokarzinom des menschlichen Colons abgeleitet ist (Western Blotting),
 - er erkennt Ratten-Villin in der Immunocytochemie (mit Formaldehyd fixierte Gefrierschnitte (Kryostat) von Ratten-Darmschleimhaut),
 - er wird durch das am 29. April 1985 unter der Hinterlegungsnummer I-440 beim C.N.C.M. hinterlegte Hybridom produziert.

**3.** Verfahren zur Herstellung eines monoklonalen Antikörpers nach Anspruch 2, **dadurch gekennzeichnet,** daß der ausgewählte Antikörper eine antigene Stelle erkennt, die in der C-terminalen Sequenz (779-854) oder dem "Kopfteil" von Hühner-Villin der Formel enthalten ist:

<div align="center">

1                                         10  11

Val-Phe-Thr-Ala-Thr-Thr-Thr-Leu-Val-Pro-Thr-Lys-Leu-Glu-Thr-Phe-Pro-

20                                  30  31

Leu-Asp-Val-Leu-Val-Asn-Thr-Ala-Ala-Glu-Asp-Leu-Pro-Arg-Gly-Val-Asp-

40  41                        50  51

Pro-Ser-Arg-Lys-Glu-Asn-His-Leu-Ser-Asp-Glu-Asp-Phe-Lys-Ala-Val-Phe-

60  61                        70

Gly-Met-Thr-Arg-Ser-Ala-Asn-Leu-Pro-Leu-Trp-Lys-Gln-Gln-Asn-Leu-Lys-

71

Lys-Glu-Glu-Lys-Gly-Leu-Phe

</div>

wobei dieser monoklonale Antikörper in der Lage ist, diesen "Kopfteil" zu erkennen, ob dieser isoliert oder im Inneren des im Hühner-Villin vorliegenden Peptids mit 51 kd vorhanden ist.

4. Verfahren zur Herstellung eines Hybridoms, der die nach Anspruch 2 hergestellten monoklonalen Antikörper bildet, umfassend die Fusion von Milzzellen einer zuvor mit Schweine-Villin immunisierten Maus mit Myelomzellen und Gewinnung des am 29. April 1985 unter der Hinterlegungsnummer I-440 beim C.N.C.M. hinterlegten Hybridoms.

5. Verfahren zur Herstellung biologisch reinen Human-Villins, **dadurch gekennzeichent,** daß es die folgenden Schritte umfaßt:
   - Behandlung eines Lysats von Human-Enterozyten mit einer wäßrigen Lösung, die ein geeignetes Detergens enthält, und Gewinnen einer das Human-Villin enthaltenden Lösung,
   - Überführen der das Human-Villin enthaltenden Lösung über eine Affinitätssäule, die einen monoklonalen Antikörper trägt, wie den gemäß Anspruch 2 oder 3 erhaltenen, unter Bedingungen, welche die selektive Bindung des Human-Villins durch den Antikörper ermöglichen,
   - Gewinnung des Human-Villins durch Dissoziierung des Antigen-Antikörper-Komplexes, wobei das Villin bei der Untersuchung der elektrophoretischen Wanderung auf Polyacrylamidgel nur eine einzige Molekulargewichtsbande von 95 kD ergibt.

6. Verfahren nach Anspruch 1, **dadurch gekennzeichnet,** daß das Reagens, welches das Gen erkennt, das für Human-Villin codiert, durch eine DNA gebildet ist, deren Sequenz einen Bereich aufweist, der für eine Aminosäuresequenz von Human-Villin codiert, insbesondere den COOH-terminalen Bereich.

7. Verfahren zur Herstellung der Nucleinsäure, die der vollständigen mRNA des Human-Villins oder einem DNA-Fragment entspricht, dessen Nucleotidsequenz einen Bereich aufweist, der für eine Aminosäuresequenz des Human-Villins codiert, welcher dazu geeignet ist, sich spezifisch mit einer für Villin codierenden Nucleotidsequenz zu hybridisieren, **dadurch gekennzeichnet,** daß es die folgenden Schritte umfaßt:
   - Extraktion von RNA ausgehend von einer Human-Villin exprimierenden Zellinie,
   - Gewinnung der mRNA und gegebenenfalls
   - Gewinnung der DNA ausgehend von dieser mRNA.

8. Verfahren zur Herstellung eines cDNA-Fragments, **dadurch gekennzeichnet,** daß man
   - eine cDNA-Bank bildet,
   - ein cDNA-Fragment selektioniert, welches mindestens einen Teil der Nucleotidsequenz enthält, die für Aminosäuren des COOH-Endes des Human-Villins codiert, welche Nucleotidkette und entsprechende Aminosäurekette die folgenden Sequenzen aufweisen:

```
LYS TRP SER ASN THR LYS SER TYR GLU ASP LEU LYS ALA GLU
AAG TGG AGT AAC ACC AAA TCC TAT GAG GAC CTG AAG GCG GAG


SER GLY ASN SER ARG ASP TRP SER GLN ILE THR ALA GLU VAL
TCT GGC AAC TCT AGG GAC TGG AGC CAG ATC ACT GCT GAG GTC


THR SER PRO LYS VAL ASP VAL PHE ASN ALA ASN SER ASN LEU
ACA AGC CCC AAA GTG GAC GTG TTC AAT GCT AAC AGC AAC CTC


SER SER GLY PRO LEU PRO ILE PHE PRO LEU GLU GLN LEU VAL
AGT TCT GGG CCT CTG CCC ATC TTC CCC CTG GAG CAG CTA GTG


ASN LYS PRO VAL GLU GLU LEU PRO GLU GLY VAL ASP PRO SER
AAC AAG CCT GTA GAG GAG CTC CCC GAG GGT GTG GAC CCC AGC


ARG LYS GLU GLU HIS LEU SER ILE GLU ASP PHE THR GLN ALA
AGG AAG GAG GAA CAC CTG TCC ATT GAA GAT TTC ACT CAG GCC


PHE GLY MET THR PRO ALA ALA PHE SER ALA LEU PRO ARG TRP
TIT GGG ATG ACT CCA GCT GCC TTC TCT GCT CTG CCT CGA TGG


LYS GLN GLN ASN LEU LYS LYS GLU LYS GLY LEU PHE *
AAG CAA CAA AAC CTC AAG AAA GAA AAA GGA CTA TTT TGA GAAG
AGTAGCTGTGGTTGTAAAGCAGTACCCTACCCTGATTG-
TAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAAA
```

9. Verfahren zur Herstellung einer Sonde zum Nachweis von mRNA oder DNA, die für Human-Villin codieren, und die mindestens einen Teil der C-terminalen Nucleotidsequenz enthalten, wie man sie nach dem Verfahren gemäß Anspruch 7 erhält, umfassend das Markieren dieser Sequenz mit einem radioaktiven Element oder irgendeiner anderen Gruppe, die ihre Erkennung in hybridisiertem Zustand mit dem Präparat, das die zu untersuchenden mRNA oder DNA enthält, ermöglicht.

10. Verfahren zur Herstellung der Nucleotidsequenz, die für das C-terminale Ende des Human-Villins codiert, **dadurch gekennzeichnet,** daß man
   - mit Hilfe an sich bekannter Verfahrensweisen ausgehend von einer Zellinie, die Villin exprimiert, vollständige mRNA (Messenger-RNA) herstellt;
   - eine cDNA-Bank bildet (komplementäre DNA);

- die cDNA in einen Vektor einfügt, der in der Lage ist, das durch die Einfügung codierte Protein zu exprimieren;
- mit Hilfe der erhaltenen rekombinanten Vektoren einen Bakterienstamm transformiert und dann Bedingungen einstellt, die die Expression des gewünschten Proteins in dem Bakterium ermöglichen;
- mit Hilfe von Villin erkennenden Antikörpern die rekombinanten Klone selektioniert, welche den für Villin spezifischen Klon enthalten.

11. Verfahren nach Anspruch 10, **dadurch gekennzeichnet,** daß der Aufbau der cDNA-Bank mit Hilfe eines Klonierungsvektors bewirkt wird, der dazu geeignet ist, das durch die eingefügte cDNA codierte Protein zu exprimieren, insbesondere mit Hilfe von Plasmiden des Typs pEX.

12. Verfahren nach Anspruch 10 oder 11, **dadurch gekennzeichnet,** daß man einen Bakterienstamm des Typs E. coli, insbesondere E. coli pop 2135, mit Hilfe von rekombinanten Vektoren, welche eine für das C-terminale Ende des Human-Villins codierende Nucleotidsequenz enthalten, transformiert.

13. Verfahren nach einem der Ansprüche 10 bis 12, **dadurch gekennzeichnet,** daß man ein Screening der cDNA-Bank mit Hilfe eines polyklonalen Anti-Villin-Antikörpers, wie eines gegen intaktes Schweine-Villin gerichteten polyklonalen Antikörpers, und dann mit einem oder mehreren monoklonalen Antikörpern, die in dem COOH-terminalen Bereich vorliegende Epitope erkennen, bewirkt, wobei das Screening mit Hilfe der polyklonalen Antikörper mit Vorteil von einem sekundären Screening mit Hilfe eines weiteren polyklonalen Antikörpers gefolgt wird, insbesondere mit Hilfe eines gegen das COOH-terminale Fragment des Hühner-Villins gerichteten polyklonalen Antikörpers.

14. Herstellung eines Klons, der eine dem Human-Villin entsprechende cDNA trägt, **dadurch gekennzeichnet,** daß man am Ende des Verfahrens gemäß Anspruch 10 einen Klon selektioniert, der spezifisch mit dem gegen das intakte Villin gerichteten polyklonalen Antikörper, mit dem gegen das COOH-terminale Peptid des Hühner-Villins gerichteten polyklonalen Antikörper und mit den beiden gegen die COOH-terminalen Epitope gerichteten monoklonalen Antikörpern reagiert.

33